(19) Europäisches Patentamt
European Patent Office
Office européen des brevets

(11) EP 4 119 138 A1

(12) **EUROPEAN PATENT APPLICATION**

(43) Date of publication:
**18.01.2023 Bulletin 2023/03**

(21) Application number: **21185175.3**

(22) Date of filing: **12.07.2021**

(51) International Patent Classification (IPC):
***A61K 31/343*** *(2006.01)* ***A61K 31/4418*** *(2006.01)*
***A61K 31/451*** *(2006.01)* ***A61K 31/495*** *(2006.01)*
***A61K 31/5375*** *(2006.01)* ***A61K 31/54*** *(2006.01)*
***A61P 31/14*** *(2006.01)*

(52) Cooperative Patent Classification (CPC):
**A61K 31/343; A61K 31/4418; A61K 31/451; A61K 31/495; A61K 31/5375; A61K 31/54; A61P 31/14**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Universität Hamburg**
**20148 Hamburg (DE)**

(72) Inventors:
- **MEIER, Chris**
  **21635 Jork (DE)**
- **RIJONO, Desiree**
  **20537 Hamburg (DE)**
- **FOHRMANN, Nora Constanze**
  **21035 Hamburg (DE)**

(74) Representative: **Uexküll & Stolberg Partnerschaft von Patent- und Rechtsanwälten mbB**
**Beselerstraße 4**
**22607 Hamburg (DE)**

(54) **DHODH INHIBITORS AND THEIR USE AS ANTIVIRAL AGENTS**

(57) The present invention relates to a compound, or a dimer or a pharmaceutically acceptable salt or solvate of said compound or dimer, for use in a method for the treatment of a disease, disorder or condition caused by an RNA virus, said compound having the general structure shown in Formulae I, II or III:

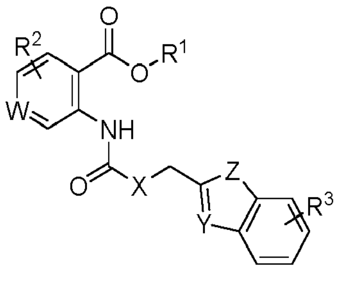

(I),

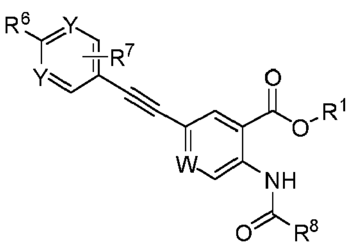

(II),

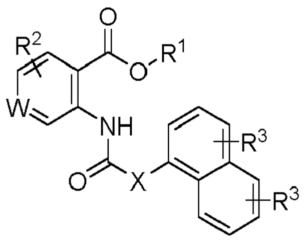

(III).

EP 4 119 138 A1

## Description

The present invention relates to anthranilic acid compounds useful as DHODH inhibitors, pharmaceutical compositions containing the compounds, and the use of the compounds and compositions in methods of treatment of a disease, disorder or condition caused by an RNA virus.

While the demand for nucleotides is covered by the salvage pathway in resting cells, fast proliferating cells, like cells of the immune system, tumor cells, or virally infected cells are highly dependent on the de *novo* nucleotide synthesis. Accordingly, the uses of inhibitors of the de *novo* nucleotide biosynthetic pathways have been investigated in the past.

Dihydroorotate dehydrogenase (DHODH) is a mitochondrial enzyme involved in pyrimidine metabolism. DHODH catalyzes the fourth step of the de *novo* synthesis of uridine monophosphate (UMP), which is afterwards converted to all other pyrimidine nucleotides. An inhibition of DHODH and thus a suppression of de *novo* synthesis of UMP leads to a downregulation of the intracellular number of pyrimidine nucleotides. This can lead to limitations in cell growth and proliferation.

Munier-Lehmann et al. (J. Med. Chem. 2013, 56, 3148-3167) report about the potential use of DHODH inhibitors in several therapeutic fields, such as the treatment of autoimmune diseases, e.g. rheumatoid arthritis or multiple sclerosis, the prophylaxis of transplant rejection, the treatment of cancer, e.g. leukemia or malignant melanoma, the treatment of viral and parasite infections, e.g. malaria, as well as in crop science.

One of the most potent DHODH inhibitors is brequinar with an $IC_{50}$ of about 10 nM. It has been investigated for cancer treatment and as an immunosuppressive drug in clinical trials. However, due to a narrow therapeutic window and inconsistent pharmacokinetics the further development was cancelled.

Another well described DHODH inhibitor is teriflunomide that derives from the prodrug leflunomide. It is the first approved DHODH inhibitor for the treatment of rheumatoid arthritis and is also used against multiple sclerosis. Disadvantages of this drug are a long plasma half-life and liver toxicity.

Chen et al. (Transplant Immunology 23 (2010) 180 -184) describe the use of two DHODH inhibitor compounds, ABR-222417 and ABR-224050, as immunosuppressive agent. In particular, ABR-222417 is reported to result in a marked increase in graft survival time when screened in a low-responder heart allograft transplantation model in rats.

WO 2005/075410 discloses the use of certain DHODH inhibitor compounds for the treatment of autoimmune diseases, inflammatory diseases, organ transplant rejection and malignant neoplasia.

Compared to autoimmune diseases and oncology, virology is a rather novel field of application of DHODH inhibitors. US 2014/0080768 A1 and WO 2009/153043 A1 generally mention the potential use of DHODH inhibitors for various diseases and disorders including some viral diseases, besides autoimmune diseases, immune and inflammatory diseases, cancer, destructive bone disorders and others.

As regards DNA-viruses, Marschall et al. (Antiviral Res. 2013, 100, 640-648) focused on the *in vivo* antiviral efficacy of 3-(2,3,5,6-tetrafluoro-3'-trifluoromethoxy-biphenyl-4-ylcarbomyl)thiophene-2-carboxylic acid by inhibition of cytomegalovirus replication in mice. With respect to RNA-viruses, Wang et al. (J. Virol. 85 (2011), 6548-6556) investigated the inhibition of dengue virus (DENV) by NITD-982. While this compound demonstrated some *in vitro* potency, it did not show any efficacy in a mouse model.

Until now no DHODH inhibitor is used in antiviral therapy.

Therefore, there is still a need for compounds overcoming the above-mentioned drawbacks and problems. Accordingly, it is an object of the present invention to provide compounds which are suitable as antiviral agents and especially suitable to treat diseases, disorders or conditions caused by RNA viruses. In particular, it would be desirable to provide compounds having a high antiviral efficacy and at the same time a broad therapeutic window, i.e. a broad range of doses at which the therapeutic benefit is achieved without resulting in unacceptable side-effects or toxicity. Moreover, it would be desirable that the compounds are characterized by acceptable pharmacokinetic parameters, like ADME (Absorption, Distribution, Metabolism, and Excretion) properties. In addition, the compounds should be obtainable easily and in high yields avoiding cumbersome routes of synthesis.

It has surprisingly been found that the above problems are solved by the compounds defined below and in the appended claims.

Accordingly, in a first aspect the present invention relates to a compound, or a dimer or a pharmaceutically acceptable salt or solvate of said compound or dimer, for use in a method for the treatment of a disease, disorder or condition caused by an RNA virus, said compound having the general structure shown in Formula I:

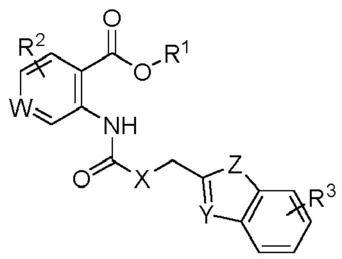

(I)

wherein:

W is C or N, and preferably is C;

$R^1$ is H, alkyl, cycloalkyl, heterocyclyl, -C(O)-alkyl or a pharmaceutically acceptable cation, wherein the alkyl or -C(0)-alkyl can be unsubstituted or optionally substituted with one or more moieties which can be the same or different, each moiety being independently selected from the group consisting of -OC(O)-alkyl, -OC(O)O-alkyl, heterocyclyl, aryl and heteroaryl,
or optionally the -C(O)-O-$R^1$-group is joined to the -NH-group or -X-group shown in Formula (I) to form together with the W-containing aromatic ring shown in Formula (I) a hetero ring system;

$R^2$ is one or more substituents independently selected from the group consisting of H, alkyl, alkenyl, alkynyl, aryl, alkoxy, aryloxy, halogen, haloalkyl, cycloalkyl, halocycloalkyl, heterocyclyl, hydroxyalkyl and -$NO_2$, wherein each of said alkyl, alkenyl, alkynyl, aryl, alkoxy, cycloalkyl, halocycloalkyl, heterocyclyl and aryloxy can be unsubstituted or optionally substituted with one or more moieties which can be the same or different, wherein each moiety is preferably independently selected from the group consisting of hydroxyl, halogen, alkyl, haloalkyl, aryl, haloaryl, alkylaryl, arylalkyl, cycloalkyl, aryloxy, alkoxy substituted with aryl, alkyl substituted with heterocyclyl, aryl substituted with haloaklkyl, aryl substituted with cycloalkyl, aryl substituted with arylalkyl, aryl substituted with alkoxy, aryl substituted with aryloxy, aryl substituted with -O-arylalkyl, aryl substituted with aryl, aryloxy, aryloxy substituted with alkyl, aryloxy substituted with cycloalkyl and aryloxy substituted with arylalkyl,
or optionally $R^2$ represents two substituents which are joined to form together with the aromatic ring shown in Formula I a substituted or unsubstituted ring or hetero ring system,
or optionally at least one of $R^2$ is joined to the -NH-group or -X-group shown in Formula (I) to form together with the W-containing aromatic ring shown in Formula (I) a hetero ring system;

$R^3$ is one or more substituents independently selected from the group consisting of H, alkyl, halogen, haloalkyl, cycloalkyl, halocycloalkyl, heterocyclyl, alkoxy, aryl and arylalkyl, wherein each of said alkyl, haloalkyl, cycloalkyl, halocycloalkyl, heterocyclyl, alkoxy, aryl and arylalkyl can be unsubstituted or optionally substituted with one or more moieties which can be the same or different, wherein each moiety is preferably independently selected from the group consisting of hydroxyl, halogen, alkyl, haloalkyl, aryl, haloaryl, alkylaryl, arylalkyl, cycloalkyl, aryloxy, alkoxy substituted with aryl, alkyl substituted with heterocyclyl, aryl substituted with haloaklkyl, aryl substituted with cycloalkyl, aryl substituted with arylalkyl, aryl substituted with alkoxy, aryl substituted with aryloxy, aryl substituted with -O-arylalkyl, aryl substituted with aryl, aryloxy, aryloxy substituted with alkyl, aryloxy substituted with cycloalkyl and aryloxy substituted with arylalkyl;

X is O or NH, and preferably is NH;

Y is $CR^4$ or N, and preferably is $CR^4$;

Z is O, S, NH or $CR^5_2$, and preferably is O;

$R^4$ is H, halogen, alkyl, haloalkyl, cycloalkyl, halocycloalkyl, heterocyclyl, alkoxy or acetyl, and preferably is H; and

$R^5$ is independently selected from the group consisting of H, halogen, alkyl, haloalkyl, cycloalkyl, halocycloalkyl, heterocyclyl, alkoxy and acetyl, and preferably is H.

It has been found that the above-defined compounds have a high antiviral efficacy. In particular, the compounds have been found to have a high antiviral activity against a wide spectrum of viruses, especially a wide spectrum of RNA

viruses. Hence, the compounds have been found to be promising candidates for *in vivo* applications as broad-spectrum antiviral drug.

Furthermore, the above-defined compounds have been found to act by inhibition of a host enzyme. This brings several advantages compared to inhibitors targeting viral enzymes, such as (i) a higher barrier to resistance, (ii) a broad coverage of different viruses that goes along with a preventative preparation for new emerging virus infections, and (iii) allowing antiviral therapy of virus infections without druggable viral targets. In particular, the above-defined compounds have been found to act by inhibition of the host enzyme DHODH. The DHODH sets itself apart from other cellular targets by the fact that the host cell is able to overcome its temporary inhibition by the steady supply of pyrimidine nucleotides for resting cells via salvage pathway.

Besides showing a strong antiviral activity and strong inhibition of hDHODH, the above-defined compounds show a high metabolic stability with no cleavage at the amide group and very reduced or even no hydroxylation. A good membrane permeability and good plasma stability are shown by the compounds of the invention as well. This underscores their position as promising candidates for further *in vivo* pharmacokinetic studies.

In a preferred embodiment, the compound to be used according to the invention is represented by formula (I) and W is C. Accordingly, the monoaromatic ring shown in Formula I is preferably a phenyl ring and the compounds to be used according to the invention are anthranilic acid derivatives.

In a preferred embodiment, the compound to be used according to the invention is represented by formula (I) and $R^1$ is H. Thus, the compound is preferably present as a free anthranilic acid. However, under physiological conditions the free acid is deprotonated resulting in a negatively charged carboxylate. In order to mask this negative charge under physiological conditions, prodrug moieties can be installed at the $R^1$ position to enable penetration through the cell membrane and allow the release of the active carboxylic acid inside the cell.

Accordingly, besides H, $R^1$ can be a pharmaceutically acceptable cation or alkyl, cycloalkyl, heterocyclyl or -C(O)-alkyl, wherein the alkyl or -C(O)-alkyl can be unsubstituted or optionally substituted with one or more moieties which can be the same or different, each moiety being independently selected from the group consisting of -OC(O)-alkyl, -OC(O)O-alkyl, heterocyclyl, aryl and heteroaryl. For example, $R^1$ can be - C(O)-alkyl to form an anhydride prodrug moiety. Moreover, $R^1$ can be alkyl substituted with -OC(O)O-alkyl to form an alkoxycarbonyloxyalkyl (POC) moiety. Furthermore, $R^1$ can be alkyl substituted with aryl which in turn is substituted with an -O-C(O)-alkyl group to form an acyloxybenzyl moiety.

In one embodiment, $R^1$ is selected from the group consisting of H,

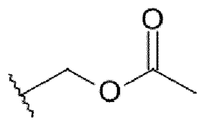

,

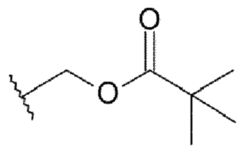

,

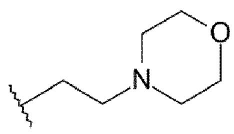

,

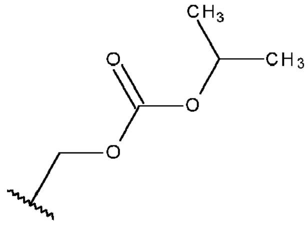

,

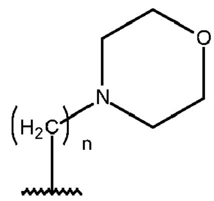

,

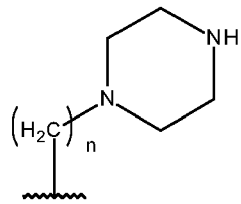

,

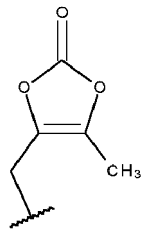

,

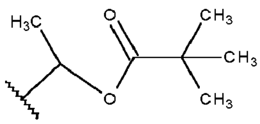

,

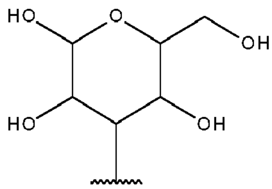

or

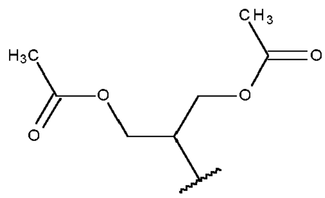

,

and preferably is H.

In a further embodiment, $R^1$ is selected from the group consisting of H,

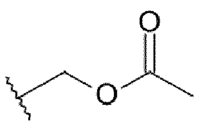

,

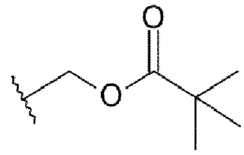

and

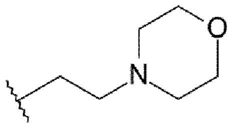

,

and preferably is H.

In a further embodiment, the -C(O)-O-$R^1$-group can be joined to the -NH-group or -X-group shown in Formula (I) to form together with the monoaromatic ring shown in Formula (I) a hetero ring system. An example of such a hetero ring system is a quinazolinedione, such as

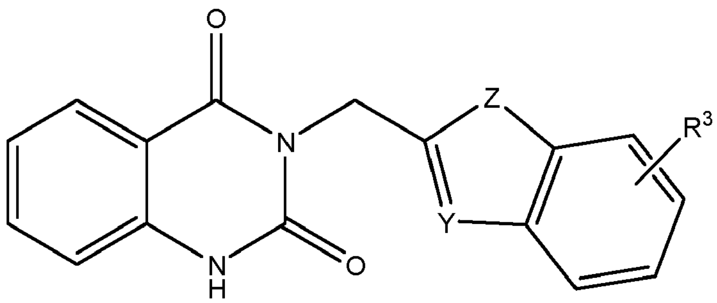

Furthermore, the compounds to be used according to the invention are characterized in that the aromatic ring of the anthranilic core is substituted by one or more $R^2$ substituents. In a one embodiment, $R^2$ is a substituent, in particular one substituent, as defined above.

In a preferred embodiment, $R^2$ is H.

In another embodiment, $R^2$ is aryl, preferably phenyl, which can be unsubstituted or optionally substituted with one or more moieties which can be the same or different, each moiety being independently selected from the group consisting of alkyl, aryl, halogen, haloaryl, alkylaryl, arylalkyl, cycloalkyl, aryloxy, alkoxy substituted with aryl, alkyl substituted with heterocyclyl.

In another embodiment, $R^2$ is

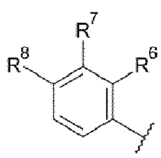

,

wherein

$R^6$ is H; halogen, preferably F; or aryl, preferably phenyl;

$R^7$ is H; halogen, preferably F; alkyl, preferably methyl; or aryl, preferably phenyl; and

$R^8$ is H; cycloalkyl, preferably cyclohexyl; aryl, preferably phenyl; haloaryl, preferably 4-F-phenyl; arylalkyl, preferably 4-ethyl-phenyl, 4-pentyl-phenyl; alkylaryl, preferably benzyl; aryloxy, preferably phenyloxy; arylalkoxy, preferably benzyloxy; or heterocyclylalkyl, preferably morpholinomethyl.

In another embodiment, $R^2$ is

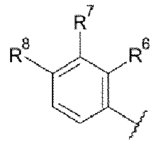,

wherein $R^6$, $R^7$ and $R^8$ are selected as shown in the following table:

| $R^6$ | $R^7$ | $R^8$ |
|---|---|---|
| H | H | Ph |
| Ph | H | H |
| H | Ph | H |
| H | F | Ph |
| H | H | 4-F-Ph |
| F | H | 4-Et-Ph |
| H | H | 4-Pentyl-Ph |
| H | H | 4-Benzyl |
| H | H | Cyclohexyl |
| H | H | OPh |
| H | $CH_3$ | $OCH_2Ph$ |
| H | F | $OCH_2Ph$ |
| H | H | Morpholinomethyl |

In another embodiment, $R^2$ is alkynyl, preferably ethynyl, which can be unsubstituted or optionally substituted with a moiety selected from the group consisting of aryl, aryl substituted with alkyl, aryl substituted with haloaklkyl, aryl substituted with cycloalkyl, aryl substituted with arylalkyl, aryl substituted with alkoxy, aryl substituted with aryloxy, aryl substituted with -O-arylalkyl, aryl substituted with aryl, aryloxy, aryloxy substituted with alkyl, aryloxy substituted with cycloalkyl and aryloxy substituted with arylalkyl.

For instance, $R^2$ is alkynyl, preferably ethynyl, which is substituted with a moiety selected from the group consisting of phenyl; phenyl substituted with 4-haloalkyl like 4-$CF_3$; phenyl substituted with 4-alkyl like 4-$C_4H_9$ or 4-$C_6H_{13}$; phenyl substituted with 4-alkoxy like 4-ethoxy or 4-pentoxy; phenyl substituted with 4-aryloxy like 4-phenyloxy; phenyl substituted with arylalkoxy like 4-benzyloxy; phenyl substituted with 4-aryl like 4-phenyl; phenyl substituted with 4-cycloalkyl like 4-cyclohexyl; phenyl substituted with 4-arylalkyl like 4-benzyl; and alkyl, preferably methyl or butyl, which is substituted with aryloxy, preferably phenyloxy, which in turn is substituted with 2-alkyl, like 2-sec-butyl, 2-cycloalkyl, like 2-cyclohexyl, or 2-arylalkyl, like 2-benzyl.

In another embodiment, $R^2$ is alkynyl, preferably ethynyl, which is substituted with a moiety selected from the group consisting of

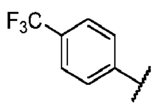, 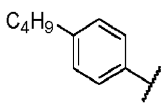, 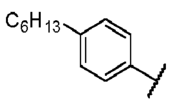, 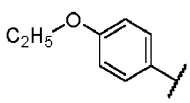, 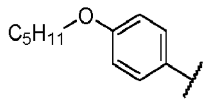,

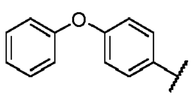, 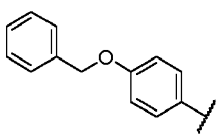, 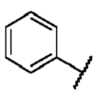, 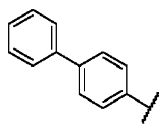, 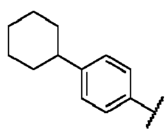, 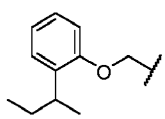,

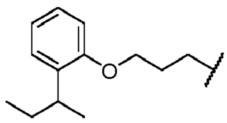, 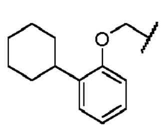, 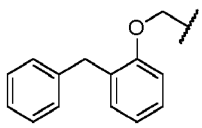, and 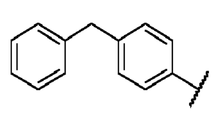.

In another embodiment, $R^2$ is alkynyl, preferably ethynyl, which is substituted with the follwing moiety:

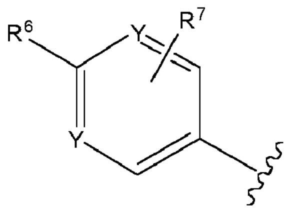

wherein

Y is independently selected from $CR^4$ and N, and preferably is $CR^4$;

$R^4$ is H, halogen, alkyl, haloalkyl, cycloalkyl, halocycloalkyl, heterocyclyl, alkoxy or acetyl, and preferably is H;

$R^6$ is heterocyclyl or heterocyclylalkyl, wherein the heterocyclyl and heterocyclylalkyl can be unsubstituted or substituted with one or more ring system substituents which can be the same or different; and

$R^7$ is one or more substituents independently selected from the group consisting of H, halogen, alkyl, haloalkyl, alkoxy, - C(O)-alkyl, -C(O)-haloalkyl, $-NH_2$, -NH-alkyl and $-N(alkyl)_2$.

Particularly suitable $R^2$-groups of this embodiment are described in further detail below in connection with the second aspect of the invention.

It is particularly preferred that $R^2$ is a rather small, i.e. a sterically undemanding, substituent, such as H; alkyl, preferably 5-alkyl like 5-methyl or 5-tBu; halogen, wherein halogen is preferably selected from F, Cl and Br, and/or wherein halogen is preferably 5-halogen like 5-Br or 5-F or 4-halogen like 4-F; alkoxy, preferably 5-alkoxy like 5-methoxy or 4-alkoxy like 4-methoxy; haloalkyl, preferably 5-$CF_3$; $NO_2$, preferably 5-$NO_2$; or aryl, preferably phenyl or biphenyl like 5-phenyl or 5-biphenyl. Most preferably, all $R^2$ are H.

In another embodiment, $R^2$ represents two substituents which are joined to form together with the monoaromatic ring shown in Formula (I) a ring system or a hetero ring system. The ring system and the hetero ring system can be unsubstituted or optionally substituted, e.g. by a ring system substituent as defined herein. In particular, the ring system can comprise an aryl, such as e.g. a phenyl, annelated to the aromatic ring of Formula (I). In this embodiment the phenyl ring of the anthranilic core of the compounds of Formula (I) and the phenyl ring annelated to it form a polycylic aromatic hydrocarbon, such as e.g. a naphthalene. In a further embodiment, the hetero ring system formed by joining two $R^2$ substituents together with the monoaromatic ring shown in Formula (I) is

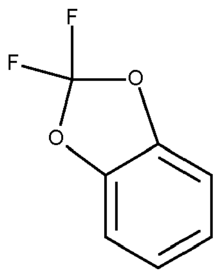

or

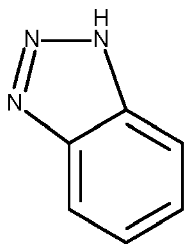

.

In a further embodiment, at least one of $R^2$ is joined to the -NH-group or -X-group to form together with the monoaromatic ring shown in Formula (I) a hetero ring system. An example of such a hetero ring system is a quinazolinedione.

Furthermore, it is preferred that the group X in formula (I) is NH.

In addition, it is preferred that the group Y in formula (I) is $CR^4$, with $R^4$ being H;
Furthermore, it is preferred that the group Z in formula (I) is O.

Moreover, it is preferred that the group $R^3$ in formula (I) is one or more substituents independently selected from the group consisting of H, alkyl, halogen and haloalkyl. In particular, the alkyl can be 2-methylbutan-2-yl, the halogen can be F, and/or the haloalkyl can be trifluoromethyl.

In another preferred embodiment, the compound of the first aspect of the invention can have the general

structure shown in Formula Ia or Ib:

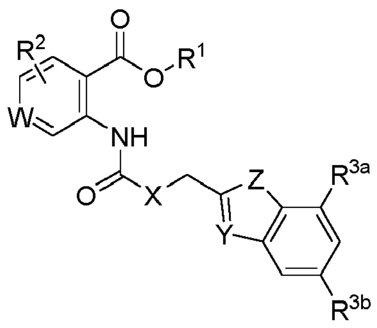

(Ia)

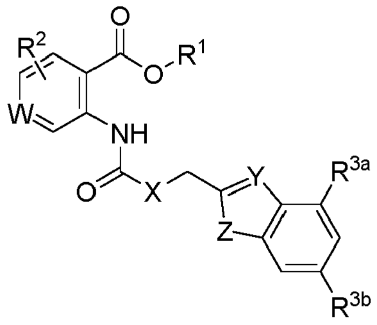

(Ib)

wherein:

$R^{3a}$ is alkyl or haloalkyl; and

$R^{3b}$ is H or halogen, wherein the halogen is preferably F.

In this embodiment, the alkyl is preferably 2-methylbutan-2-yl and the haloalkyl is preferably trifluoromethyl.

In a particularly preferred embodiment, the compound according to the first aspect of the invention has one of the following structures:

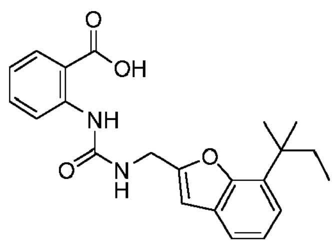

,

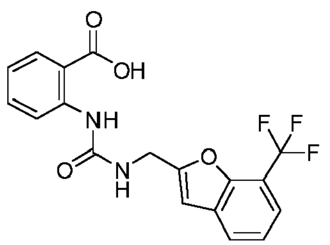

or

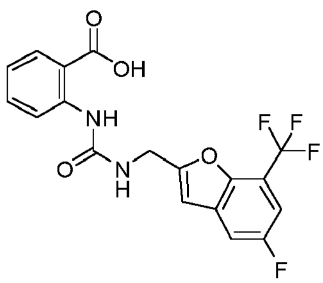

.

In a second aspect the present invention relates to a compound, or a dimer or a pharmaceutically acceptable salt or solvate of said compound or dimer, for use in a method for the treatment of a disease, disorder or condition caused by an RNA virus, said compound having the general structure shown in Formula II:

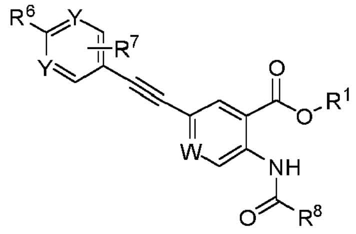

(II)

wherein:

W is C or N, and preferably is C;

$R^1$ is H, alkyl, cycloalkyl, heterocyclyl, -C(O)-alkyl or a pharmaceutically acceptable cation, wherein the alkyl or -C(0)-alkyl can be unsubstituted or optionally substituted with one or more moieties which can be the same or different, each moiety being independently selected from the group consisting of -OC(O)-alkyl, -OC(O)O-alkyl, heterocyclyl, aryl and heteroaryl;

Y is independently selected from $CR^4$ and N, and preferably is $CR^4$;

$R^4$ is H, halogen, alkyl, haloalkyl, cycloalkyl, halocycloalkyl, heterocyclyl, alkoxy or acetyl, and preferably is H;

$R^6$ is heterocyclyl or heterocyclylalkyl, wherein the heterocyclyl and heterocyclylalkyl can be unsubstituted or substituted with one or more ring system substituents which can be the same or different and are described below;

$R^7$ is one or more substituents independently selected from the group consisting of H, halogen, alkyl, haloalkyl, alkoxy, - C(O)-alkyl, -C(O)-haloalkyl, $-NH_2$, -NH-alkyl and $-N(alkyl)_2$; and

$R^8$ is -C(O)-alkyl, -C(O)O-alkyl, -C(O)NH-alkyl, -C(O)-cycloalkyl, -C(O)O-cycloalkyl, -C(O)NH-cycloalkyl, -C(O)-aryl, -C(O)O-aryl, -C(O)NH-aryl, -C(O)-heteroaryl, -C(O)O-heteroaryl, -C(O)NH-heteroaryl, aryl substituted with $R^{10}$, heteroaryl substituted with $R^{10}$, $-S(O_2)-R^{11}$ or

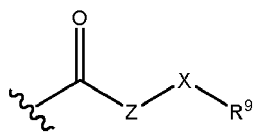

;

wherein

Z is $-(CH_2)_n-$, $-O-(CH_2)_n-$, $-NH-(CH_2)_n-$, $-(CH_2)_p-L-(CH_2)_q-$, $-O-(CH_2)_p-L-(CH_2)_q-$ or -NH- $(CH_2)p-L-(CH_2)_q-$,

n is an integer from 1 to 6;

p and q are integers independently selected from 0 to 6;

L is a linking group selected from the group consisting of heteroaryl, aryl, heterocyclyl and cycloalkyl;

X is O, S, NH, $CH_2$, S(O) or C(0), and preferably is O, S or NH;

$R^9$ is aryl or heteroaryl, wherein said aryl or heteroaryl can be unsubstituted or optionally substituted with one or more moieties which can be the same or different, each moiety being independently selected from the group consisting of H, halogen, alkyl, cycloalkyl, heterocyclyl, aryl, heteroaryl, alkoxy, aryloxy, arylalkyl, alkylaryl, haloaryl, haloalkylaryl, haloalkyl and trialkylsilyl;

$R^{10}$ is aryloxy or arylalkyl or optionally $R^{10}$ represents two substituents linked to each other to form together with the aryl or heteroaryl a polycyclic ring system, wherein preferably the polycyclic ring system is a naphthalene or fluorene; and

$R^{11}$ is alkyl, cycloalkyl or -Z-X-$R^9$.

It has been found that also the above-defined compounds of the second aspect have a high antiviral efficacy and strong inhibition of the hDHODH host enzyme. Thus, also the compounds of the second aspect of the invention have been found to be promising candidates for *in vivo* applications as broad-spectrum antiviral drug.

In addition, it has surprisingly been found that the compounds of the second aspect are less vulnerable for metabolism compared to the inhibitors described in WO 2020/225330. Without wishing to be bound by any theory, it is believed that due to the heterocyclyl or heterocyclylalkyl substituent $R^6$ polarity is added and the compounds are less lipophilic which makes them less vulnerable for metabolism.

In a preferred embodiment of formula (II), $R^6$ is selected from the group consisting of

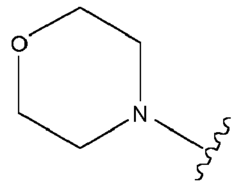

,

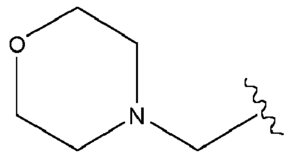

,

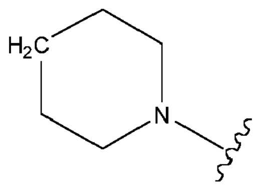

,

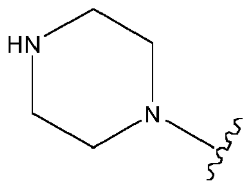

,

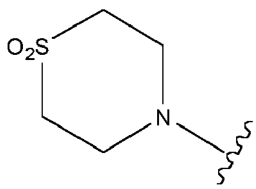

and

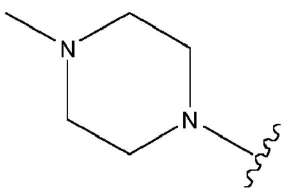

and

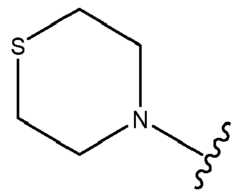

,

and preferably is

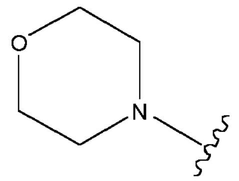

.

Furthermore, in a preferred embodiment of formula (II), $R^7$ is one or more substituents independently selected from the group consisting of H, halogen, alkyl, haloalkyl, -C(O)-alkyl, - C(O)-haloalkyl, -NH-alkyl and -N(alkyl)$_2$. Preferably, the alkyl in the above $R^7$ groups is methyl or ethyl. Moreover, if $R^7$ is halogen, it is preferaby F.

In a particularly preferred embodiment, the compound according to the second aspect of the invention has one of the following structures:

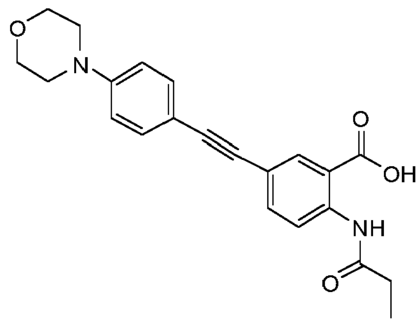

,

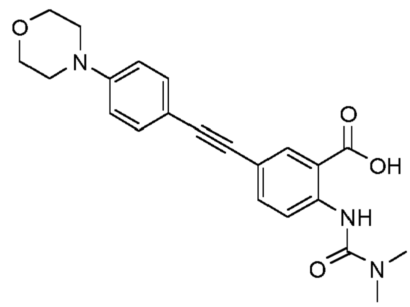

,

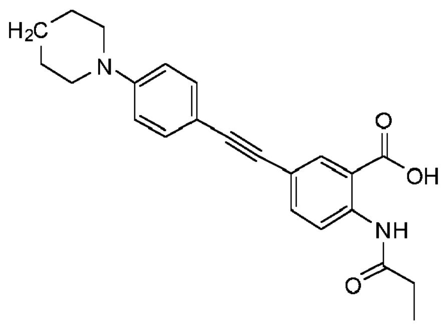

,

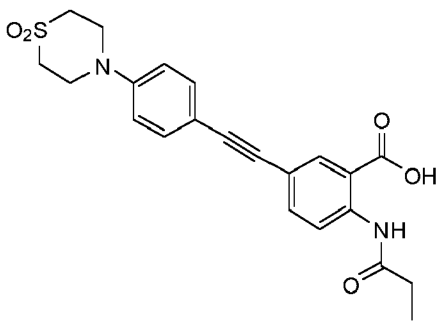

,

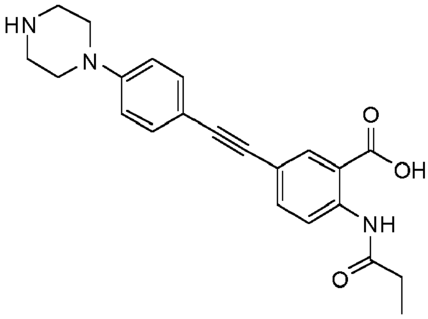 , 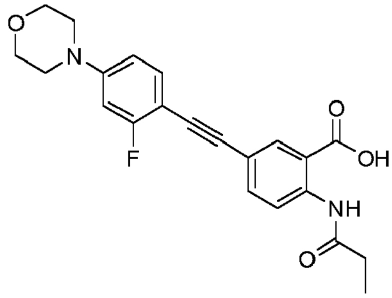 ,

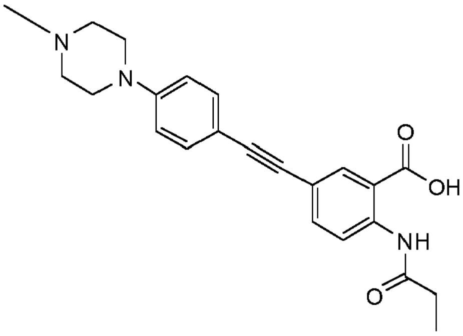 , 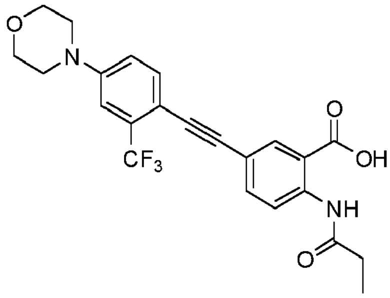 ,

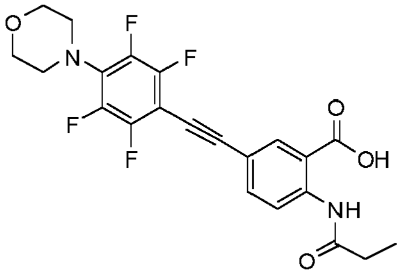 , 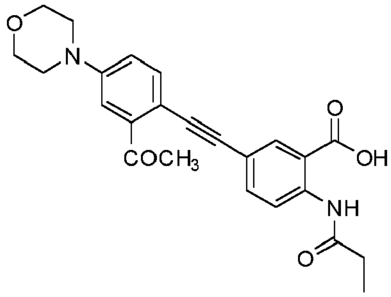 ,

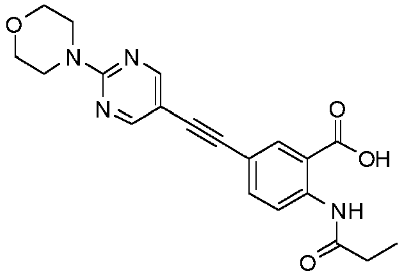 , 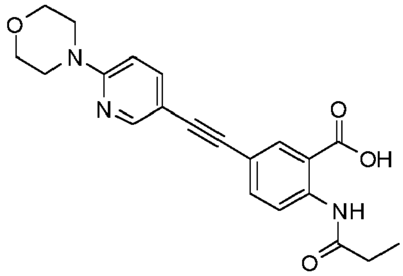 or

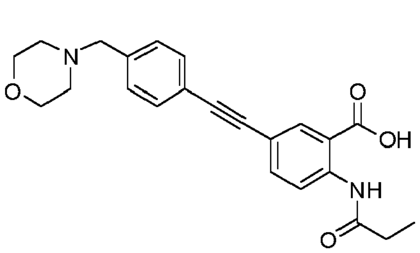 .

In a third aspect the present invention relates to a compound, or a dimer or a pharmaceutically acceptable salt or solvate of said compound or dimer, for use in a method for the treatment of a disease, disorder or condition caused

by an RNA virus, said compound having the general structure shown in Formula III:

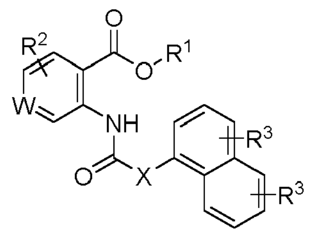

(III)

wherein:

W is N;

$R^1$ is H, alkyl, cycloalkyl, heterocyclyl, -C(O)-alkyl or a pharmaceutically acceptable cation, wherein the alkyl or -C(O)-alkyl can be unsubstituted or optionally substituted with one or more moieties which can be the same or different, each moiety being independently selected from the group consisting of -OC(O)-alkyl, -OC(O)O-alkyl, heterocyclyl, aryl and heteroaryl,
or optionally the -C(O)-O-$R^1$-group is joined to the -NH-group or -X-group shown in Formula (III) to form together with the W-containing aromatic ring shown in Formula (III) a hetero ring system;

$R^2$ is one or more substituents independently selected from the group consisting of H, alkyl, alkenyl, alkynyl, aryl, alkoxy, aryloxy, halogen, haloalkyl, cycloalkyl, halocycloalkyl, heterocyclyl, hydroxyalkyl and -$NO_2$, wherein each of said alkyl, alkenyl, alkynyl, aryl, alkoxy, cycloalkyl, halocycloalkyl, heterocyclyl and aryloxy can be unsubstituted or optionally substituted with one or more moieties which can be the same or different, wherein each moiety is preferably independently selected from the group consisting of hydroxyl, halogen, alkyl, haloalkyl, aryl, haloaryl, alkylaryl, arylalkyl, cycloalkyl, aryloxy, alkoxy substituted with aryl, alkyl substituted with heterocyclyl, aryl substituted with haloaklkyl, aryl substituted with cycloalkyl, aryl substituted with arylalkyl, aryl substituted with alkoxy, aryl substituted with aryloxy, aryl substituted with -O-arylalkyl, aryl substituted with aryl, aryloxy, aryloxy substituted with alkyl, aryloxy substituted with cycloalkyl and aryloxy substituted with arylalkyl,
or optionally $R^2$ represents two substituents which are joined to form together with the aromatic ring shown in Formula III a substituted or unsubstituted ring or hetero ring system,
or optionally at least one of $R^2$ is joined to the -NH-group or -X-group shown in Formula (III) to form together with the W-containing aromatic ring shown in Formula (III) a hetero ring system;

X is $CH_2$, O or NH, and preferably is NH; and

$R^3$ is one or more substituents independently selected from the group consisting of H, alkyl, halogen, haloalkyl, cycloalkyl, halocycloalkyl, heterocyclyl, alkoxy, aryl, arylalkyl, heteroalkyl, heteroarylalkyl, amido, carbamoyl, wherein each of said alkyl, haloalkyl, cycloalkyl, halocycloalkyl, heterocyclyl, alkoxy, aryl, arylalkyl, heteroalkyl, heteroarylalkyl, amido, and carbamoyl can be unsubstituted or optionally substituted with one or more moieties which can be the same or different, wherein each moiety is preferably independently selected from the group consisting of hydroxyl, halogen, alkyl, haloalkyl, aryl, haloaryl, alkylaryl, arylalkyl, cycloalkyl, aryloxy, alkoxy substituted with aryl, alkyl substituted with heterocyclyl, aryl substituted with haloaklkyl, aryl substituted with cycloalkyl, aryl substituted with arylalkyl, aryl substituted with alkoxy, aryl substituted with aryloxy, aryl substituted with -O-arylalkyl, aryl substituted with aryl, aryloxy, aryloxy substituted with alkyl, aryloxy substituted with cycloalkyl and aryloxy substituted with arylalkyl;

or optionally $R^3$ represents two substituents which are joined to form together with the naphthyl ring system shown in Formula III a substituted or unsubstituted ring or hetero ring system.

It has been found that also the above-defined compounds of the third aspect have a very high antiviral efficacy and strong inhibition of the hDHODH host enzyme. Thus, also the compounds of the third aspect of the invention have been found to be promising candidates for *in vivo* applications as broad-spectrum antiviral drug.
In addition, it has surprisingly been found that the compounds of the third aspect show a high metabolic stability and good membrane permeability.
In a preferred embodiment of the third aspect of the invention, the compound has the general structure shown

in Formula IIIa:

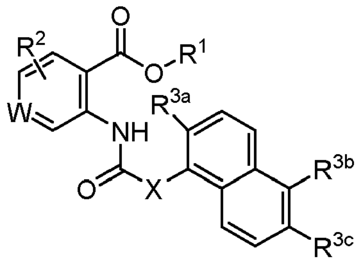
(IIIa)

wherein $R^1$, $R^2$, W and X are as defined above in formula (III);

$R^{3a}$ is H, $-CH_3$, $-CF_3$, or halogen; and

$R^{3b}$ and $R^{3c}$ are the same or different and are independently selected from the group consisting of H, halogen, alkyl, cycloalkyl, heterocyclyl, aryl, heteroaryl, alkoxy, aryloxy, arylalkyl, alkylaryl, haloaryl, haloalkylaryl, haloalkyl, trialkylsilyl and carbonyl substituents, such as amides or ureas. In a further embodiment, $R^{3b}$ and $R^{3c}$ are joined to each other to form - together with the ring they are attached to - a possibly substituted ring system.

Redoxal, a known DHODH inhibitor, is a compound synthesized via the coupling of two anthranilic acid compounds. Hence, also the compounds to be used according to the present invention can be present in the form of a dimer, i.e. two molecules having the structure of formula (I), (II) or (III) can be coupled to form a dimer. In the first and third aspect of the invention, two molecules of formula (I) or two molecules of formula (III) are coupled via substituents $R^2$ and/or $R^3$. Thus, the phenyl ring of the first anthranilic acid core is coupled via $R^2$ to $R^2$ of the phenyl ring of the second anthranilic acid core or to $R^3$ of the benzodioxole-type or naphthyl ring system of the second anthranilic acid core. In the second aspect of the invention, two molecules of formula (II) are coupled via substituents $R^6$/$R^7$ and/or $R^8$. Thus, the Y-containing monoaromatic ring of the first anthranilic acid core is coupled via $R^6$ or $R^7$ to $R^6$ or $R^7$ of the Y-containing monoaromatic ring of the second anthranilic acid core or to $R^8$ of the second anthranilic acid core. Suitable routes of synthesis to provide dimers according to the invention are known and are described below.

As used above, and throughout this disclosure, the following terms, unless otherwise indicated, shall be understood to have the following meanings:

"Alkyl" means an aliphatic hydrocarbon group which may be straight or branched and comprising about 1 to about 20 carbon atoms in the chain. Preferred alkyl groups contain about 1 to about 12 carbon atoms in the chain. More preferred alkyl groups contain about 1 to about 6 carbon atoms in the chain. Branched means that one or more lower alkyl groups such as methyl, ethyl or propyl, are attached to a linear alkyl chain.

"Lower alkyl" means a group having about 1 to about 6 carbon atoms in the chain which may be straight or branched. The term "substituted alkyl" means that the alkyl group may be substituted by one or more substituents which may be the same or different, each substituent being independently selected from the group consisting of halo, alkyl, aryl, cycloalkyl, cyano, hydroxy, alkoxy, alkylthio, amino, -NH(alkyl), -NH(cycloalkyl), $-N(alkyl)_2$, carboxy and -C(O)O-alkyl. Non-limiting examples of suitable alkyl groups include methyl, ethyl, n-propyl, isopropyl and t-butyl.

"Alkenyl" means an aliphatic hydrocarbon group containing at least one carbon-carbon double bond and which may be straight or branched and comprising about 2 to about 15 carbon atoms in the chain. Preferred alkenyl groups have about 2 to about 12 carbon atoms in the chain; and more preferably about 2 to about 4 carbon atoms in the chain. Branched means that one or more lower alkyl groups such as methyl, ethyl or propyl, are attached to a linear alkenyl chain. Non-limiting examples of suitable alkenyl groups include ethenyl and propenyl. The term "substituted alkenyl" means that the alkenyl group may be substituted by one or more substituents which may be the same or different, each substituent being independently selected from the group consisting of alkyl, aryl and cycloalkyl.

"Alkynyl" means an aliphatic hydrocarbon group containing at least one carbon-carbon triple bond and which may be straight or branched and comprising about 2 to about 15 carbon atoms in the chain. Preferred alkynyl groups have about 2 to about 12 carbon atoms in the chain; and more preferably about 2 to about 4 carbon atoms in the chain. Branched means that one or more lower alkyl groups such as methyl, ethyl or propyl, are attached to a linear alkynyl chain. Non-limiting examples of suitable alkynyl groups include ethynyl, propynyl, 2-butynyl and 3-methylbutynyl. The term "substituted alkynyl" means that the alkynyl group may be substituted by one or more substituents which may be the same or different, each substituent being independently selected from the group consisting of alkyl, aryl and cycloalkyl.

"Aryl" means an aromatic monocyclic or multicyclic ring system comprising about 6 to about 14 carbon atoms, preferably about 6 to about 10 carbon atoms. The aryl group can be optionally substituted with one or more "ring system

substituents" which may be the same or different, and are as defined herein. Non-limiting examples of suitable aryl groups include phenyl and naphthyl.

"Heteroaryl" means an aromatic monocyclic or multicyclic ring system comprising about 5 to about 14 ring atoms, preferably about 5 to about 10 ring atoms, in which one or more of the ring atoms is an element other than carbon, for example nitrogen, oxygen or sulfur, alone or in combination. Preferred heteroaryls contain about 5 to about 6 ring atoms. The "heteroaryl" can be optionally substituted by one or more "ring system substituents" which may be the same or different, and are as defined herein. The prefix aza, oxa or thia before the heteroaryl root name means that at least a nitrogen, oxygen or sulfur atom respectively, is present as a ring atom. A nitrogen atom of a heteroaryl can be optionally oxidized to the corresponding N-oxide. Non-limiting examples of suitable heteroaryls include pyridyl, pyrazinyl, furanyl, thienyl, pyrimidinyl, pyridone (including N-substituted pyridones), isoxazolyl, isothiazolyl, oxazolyl, thiazolyl, pyrazolyl, furazanyl, pyrrolyl, pyrazolyl, triazolyl, 1,2,4-thiadiazolyl, pyrazinyl, pyridazinyl, quinoxalinyl, phthalazinyl, oxindolyl, imidazo[1,2-a]pyridinyl, imidazo[2,1-b]thiazolyl, benzofurazanyl, indolyl, azaindolyl, benzimidazolyl, benzothienyl, quinolinyl, imidazolyl, thienopyridyl, quinazolinyl, thienopyrimidyl, pyrrolopyridyl, imidazopyridyl, isoquinolinyl, benzoazaindolyl, 1,2,4-triazinyl, benzothiazolyl and the like. The term "heteroaryl" also refers to partially saturated heteroaryl moieties such as, for example, tetrahydroisoquinolyl, tetrahydroquinolyl and the like.

"Aralkyl" or "arylalkyl" means an aryl-alkyl- group in which the aryl and alkyl are as previously described. Preferred aralkyls comprise a lower alkyl group. Non-limiting examples of suitable arylalkyl groups include benzyl, 2-phenethyl and naphthalenylmethyl. The bond to the parent moiety is through the alkyl.

"Alkylaryl" means an alkyl-aryl- group in which the alkyl and aryl are as previously described. Preferred alkylaryls comprise a lower alkyl group. Non-limiting example of a suitable alkylaryl group is tolyl. The bond to the parent moiety is through the aryl.

"Cycloalkyl" means a non-aromatic mono- or multicyclic ring system comprising about 3 to about 10 carbon atoms, preferably about 5 to about 10 carbon atoms. Preferred cycloalkyl rings contain about 5 to about 7 ring atoms. The cycloalkyl can be optionally substituted with one or more "ring system substituents" which may be the same or different, and are as defined herein. Non-limiting examples of suitable monocyclic cycloalkyls include cyclopropyl, cyclopentyl, cyclohexyl, cycloheptyl and the like. Non-limiting examples of suitable multicyclic cycloalkyls include 1-decalinyl, norbornyl, adamantyl and the like, as well as partially saturated species such as, for example, indanyl, tetrahydronaphthyl and the like.

"Halogen" means fluorine, chlorine, bromine, or iodine. Preferred are fluorine, chlorine and bromine.

"Ring system substituent" means a substituent attached to an aromatic or non-aromatic ring system which, for example, replaces an available hydrogen on the ring system. Ring system substituents may be the same or different, each being independently selected from the group consisting of alkyl, alkenyl, alkynyl, aryl, heteroaryl, aralkyl, alkylaryl, heteroaralkyl, heteroarylalkenyl, heteroarylalkynyl, alkylheteroaryl, hydroxy, hydroxyalkyl, alkoxy, aryloxy, aralkoxy, acyl, aroyl, halo, nitro, cyano, carboxy, alkoxycarbonyl, aryloxycarbonyl, aralkoxycarbonyl, alkylsulfonyl, arylsulfonyl, heteroarylsulfonyl, alkylthio, arylthio, heteroarylthio, aralkylthio, heteroaralkylthio, cycloalkyl, heterocyclyl, $-C(=N-CN)-NH_2$, $-C(=NH)-NH_2$, -C(=NH)-NH(alkyl), $Y_1Y_2N$-, $Y_1Y_2N$-alkyl-, $Y_1Y_2NC(O)$-, $Y_1Y_2NSO_2$- and - $SO_2NY_1Y_2$, wherein $Y_1$ and $Y_2$ can be the same or different and are independently selected from the group consisting of hydrogen, alkyl, aryl, cycloalkyl, and aralkyl. "Ring system substituent" may also mean a single moiety which simultaneously replaces two available hydrogens on two adjacent carbon atoms (one H on each carbon) on a ring system. Examples of such moiety are methylene dioxy, ethylenedioxy, $-C(CH_3)_2$- and the like which form moieties such as, for example:

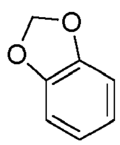
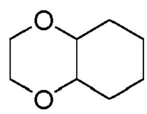
and
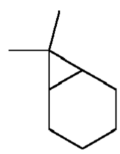
.

"Heterocyclyl" means a non-aromatic saturated monocyclic or multicyclic ring system comprising about 3 to about 10 ring atoms, preferably about 5 to about 10 ring atoms, in which one or more of the atoms in the ring system is an element other than carbon, for example nitrogen, oxygen or sulfur, alone or in combination. There are no adjacent oxygen and/or sulfur atoms present in the ring system. Preferred heterocyclyls contain about 5 to about 6 ring atoms. The prefix aza, oxa or thia before the heterocyclyl root name means that at least a nitrogen, oxygen or sulfur atom respectively is present as a ring atom. Any -NH in a heterocyclyl ring may exist protected such as, for example, as an -N(Boc), -N(CBz), -N(Tos) group and the like; such protections are also considered part of this invention. The heterocyclyl can be optionally substituted by one or more "ring system substituents" which may be the same or different, and are as defined herein. The nitrogen or sulfur atom of the heterocyclyl can be optionally oxidized to the corresponding N-oxide, S-oxide or S,S-dioxide. Non-limiting examples of suitable monocyclic heterocyclyl rings include piperidyl, pyrrolidinyl, piperazinyl, morpholinyl, thiomorpholinyl, thiazolidinyl, 1,4-dioxanyl, tetrahydrofuranyl, tetrahydrothiophenyl, lactam, lactone, and the like.

"Heteroaralkyl" means a heteroaryl-alkyl- group in which the heteroaryl and alkyl are as previously described. Preferred heteroaralkyls contain a lower alkyl group. Non-limiting examples of suitable aralkyl groups include pyridylmethyl, and quinolin-3-ylmethyl. The bond to the parent moiety is through the alkyl.

"Hydroxyalkyl" means a HO-alkyl- group in which alkyl is as previously defined. Preferred hydroxyalkyls contain lower alkyl. Non-limiting examples of suitable hydroxyalkyl groups include hydroxymethyl and 2-hydroxyethyl.

"Acyl" means an H-C(O)-, alkyl-C(O)- or cycloalkyl-C(O)-, group in which the various groups are as previously described. The bond to the parent moiety is through the carbonyl. Preferred acyls contain a lower alkyl. Non-limiting examples of suitable acyl groups include formyl, acetyl and propanoyl.

"Aroyl" means an aryl-C(O)- group in which the aryl group is as previously described. The bond to the parent moiety is through the carbonyl. Non-limiting examples of suitable groups include benzoyl and 1- naphthoyl.

"Alkoxy" means an alkyl-O- group in which the alkyl group is as previously described. Non-limiting examples of suitable alkoxy groups include methoxy, ethoxy, n-propoxy, isopropoxy and n-butoxy. The bond to the parent moiety is through the ether oxygen.

"Aryloxy" means an aryl-O- group in which the aryl group is as previously described. Non-limiting examples of suitable aryloxy groups include phenoxy and naphthoxy. The bond to the parent moiety is through the ether oxygen.

"Aralkyloxy" means an aralkyl-O- group in which the aralkyl group is as previously described. Non-limiting examples of suitable aralkyloxy groups include benzyloxy and 1- or 2-naphthalenemethoxy. The bond to the parent moiety is through the ether oxygen.

"Alkylthio" means an alkyl-S- group in which the alkyl group is as previously described. Non-limiting examples of suitable alkylthio groups include methylthio and ethylthio. The bond to the parent moiety is through the sulfur.

"Arylthio" means an aryl-S- group in which the aryl group is as previously described. Non-limiting examples of suitable arylthio groups include phenylthio and naphthylthio. The bond to the parent moiety is through the sulfur.

"Aralkylthio" means an aralkyl-S- group in which the aralkyl group is as previously described. Non-limiting example of a suitable aralkylthio group is benzylthio. The bond to the parent moiety is through the sulfur.

"Alkoxycarbonyl" means an alkyl-O-CO- group. Non-limiting examples of suitable alkoxycarbonyl groups include methoxycarbonyl and ethoxycarbonyl. The bond to the parent moiety is through the carbonyl.

"Aryloxycarbonyl" means an aryl-O-C(O)- group. Non-limiting examples of suitable aryloxycarbonyl groups include phenoxycarbonyl and naphthoxycarbonyl. The bond to the parent moiety is through the carbonyl.

"Aralkoxycarbonyl" means an aralkyl-O-C(O)- group. Non-limiting example of a suitable aralkoxycarbonyl group is benzyloxycarbonyl. The bond to the parent moiety is through the carbonyl.

"Alkylsulfonyl" means an alkyl-$S(O_2)$- group. Preferred groups are those in which the alkyl group is lower alkyl. The bond to the parent moiety is through the sulfonyl.

"Arylsulfonyl" means an aryl-$S(O_2)$- group. The bond to the parent moiety is through the sulfonyl.

The term "substituted" means that one or more hydrogens on the designated atom is replaced with a selection from the indicated group, provided that the designated atom's normal valency under the existing circumstances is not exceeded, and that the substitution results in a stable compound. Combinations of substituents and/or variables are permissible only if such combinations result in stable compounds. By "stable compound' or "stable structure" is meant a compound that is sufficiently robust to survive isolation to a useful degree of purity from a reaction mixture, and formulation into an efficacious therapeutic agent.

It should also be noted that any heteroatom with unsatisfied valences in the text, schemes, examples and Tables herein is assumed to have the hydrogen atom(s) to satisfy the valences.

When any variable (e.g., aryl, etc.) occurs more than one time in any constituent or in Formula I, II or III, its definition on each occurrence is independent of its definition at every other occurrence.

Prodrugs and solvates of the compounds of the invention are also contemplated herein. The term "prodrug", as employed herein, denotes a compound that is a drug precursor which, upon administration to a subject, undergoes chemical conversion by metabolic or chemical processes to yield a compound of Formula I, II or III or a salt and/or solvate thereof.

"Solvate" means a physical association of a compound of this invention with one or more solvent molecules. This physical association involves varying degrees of ionic and covalent bonding, including hydrogen bonding. In certain instances the solvate will be capable of isolation, for example when one or more solvent molecules are incorporated in the crystal lattice of the crystalline solid. "Solvate" encompasses both solution-phase and isolatable solvates. Non-limiting examples of suitable solvates include ethanolates, methanolates, and the like. "Hydrate" is a solvate wherein the solvent molecule is $H_2O$.

The compounds of Formula I, II or III can form salts which are also within the scope of this invention. Reference to a compound of Formula I, II or III herein is understood to include reference to salts thereof, unless otherwise indicated. The term "salt(s)", as employed herein, denotes acidic salts formed with inorganic and/or organic acids, as well as basic salts formed with inorganic and/or organic bases. In addition, when a compound of Formula I, II or III contains both a

basic moiety, such as, but not limited to a pyridine or imidazole, and an acidic moiety, such as, but not limited to a carboxylic acid, zwitterions ("inner salts") may be formed and are included within the term "salt(s)" as used herein. Pharmaceutically acceptable (i.e., non-toxic, physiologically acceptable) salts are preferred, although other salts can also be useful. Salts of the compounds of the Formula I, II or III may be formed, for example, by reacting a compound of Formula I, II or III with an amount of acid or base, such as an equivalent amount, in a medium such as one in which the salt precipitates or in an aqueous medium followed by lyophilization.

Exemplary acid addition salts include acetates, ascorbates, benzoates, benzenesulfonates, bisulfates, borates, butyrates, citrates, camphorates, camphorsulfonates, fumarates, hydrochlorides, hydrobromides, hydroiodides, lactates, maleates, methanesulfonates, naphthalenesulfonates, nitrates, oxalates, phosphates, propionates, salicylates, succinates, sulfates, tartarates, thiocyanates, toluenesulfonates (also known as tosylates,) and the like.

Exemplary basic salts include ammonium salts, alkali metal salts such as sodium, lithium, and potassium salts, alkaline earth metal salts such as calcium and magnesium salts, salts with organic bases (for example, organic amines) such as dicyclohexylamines, t-butyl amines, and salts with amino acids such as arginine, lysine and the like. Basic nitrogen-containing groups may be quarternized with agents such as lower alkyl halides (e.g. methyl, ethyl, and butyl chlorides, bromides and iodides), dialkyl sulfates (e.g. dimethyl, diethyl, and dibutyl sulfates), long chain halides (e.g. decyl, lauryl, and stearyl chlorides, bromides and iodides), aralkyl halides (e.g. benzyl and phenethyl bromides), and others.

All such acid salts and base salts are intended to be pharmaceutically acceptable salts within the scope of the invention and all acid and base salts are considered equivalent to the free forms of the corresponding compounds for purposes of the invention.

Compounds of Formula I, II or III, and salts, solvates and prodrugs thereof, may exist in their tautomeric form (for example, as an amide or imino ether). All such tautomeric forms are contemplated herein as part of the present invention.

All stereoisomers (for example, geometric isomers, optical isomers and the like) of the present compounds (including those of the salts, solvates and prodrugs of the compounds as well as the salts and solvates of the prodrugs), such as those which may exist due to asymmetric carbons on various substituents, including enantiomeric forms (which may exist even in the absence of asymmetric carbons), rotameric forms, atropisomers, and diastereomeric forms, are contemplated within the scope of this invention, as are positional isomers (such as, for example, 4-pyridyl and 3-pyridyl). Individual stereoisomers of the compounds of the invention may, for example, be substantially free of other isomers, or may be admixed, for example, as racemates or with all other, or other selected, stereoisomers.

The compounds of Formula I, II and III have pharmacological properties. In particular, the compounds of Formula I, II and III have found to be inhibitors of DHODH. Moreover, it has been found that the compounds of Formula I, II and III have a very high antiviral efficacy against various RNA viruses in an up to one-digit nanomolar range. In a preferred embodiment, the compounds to be used according to the invention are characterized by an $IC_{50}$ of less than 1.0 μM or 0.7 μM, preferably less than 0.3 μM, more preferably less than 0.1 μM, like less than 0.08 μM or less than 0.05 μM or less than 0.04 μM or less than 0.02 μM, wherein the $IC_{50}$ values are generated in an assay using LASV, EBOV or CCHFV using the assay described in the examples herein. Moreover, the compounds are characterized by a low cytotoxicity, such as a $CC_{50}$ of more than 1 μM, preferably more than 8 μM, more preferably more than 15 μM, like more than 30 μM or more than 50 μM or more than 70 μM or more than 100 μM. Hence, the compounds of Formula I, II or III are expected to be useful in the therapy of viral diseases, in particular in the treatment of treatment of a disease, disorder or condition caused by an RNA virus.

As used herein, the term "RNA virus" refers to a virus that has RNA (ribonucleic acid) as its genetic material. This nucleic acid is usually single-stranded RNA (ssRNA) but may be double-stranded RNA (dsRNA). In particular, the RNA virus is a virus that belongs to *Group III, Group IV* or *Group V* of the Baltimore classification system of classifying viruses.

Preferably, the RNA virus which can be affected by the compounds of Formula I is selected from the group consisting of bunya viruses including Toscana virus (TOSV), hazara virus (HAZV), tahyna virus (TAHV), rift valley fever virus (RVFV), Lassa virus (LSAV), Punta Toro phlebovirus (PTV) and Crimean-Congo hemorrhagic fever orthonairovirus (CCHFV); flavi viruses including yellow fever virus (YFV), dengue virus (DENV), tick-borne encephalitis virus (TBEV), zika virus (ZIKV) and Hepatitis C virus (HCV); toga viruses including venezuelan equine encephalitis virus (VEEV), Sindbis virus (SINV) and Chikungunya virus (CHIKV); mononegaviruses including Ebola virus (EBOV), Marburg virus (MARV), Human parainfluenza virus 3 (HPIV-3), Nipah virus (NiV) and Vesicular stomatitis virus (VSV); picorna viruses including coxsackievirus (CV); nidoviruses including corona viruses such as Severe acute respiratory syndrome-related coronavirus (SARS-CoV), Severe acute respiratory syndrome-related coronavirus 2 (SARS-CoV-2) and Middle-East respiratory syndrome-related coronavirus (MERS-CoV); influenza viruses and reoviruses including reovirus type 1 (Reo-1).

Hence, also disclosed is a method of treating a mammal (*e.g.*, human) having a disease or condition associated with a virus, in particular RNA virus, by administering a therapeutically effective amount of at least one compound of

Formula I, or a pharmaceutically acceptable salt or solvate of said compound to the mammal.

Moreover, the compounds according to the invention are suitable to be used for the treatment of autoimmune diseases, e.g. rheumatoid arthritis or multiple sclerosis, the prophylaxis of transplant rejection, the treatment of cancer, e.g. leukemia or malignant melanoma, the treatment of viral and parasite infections, e.g. malaria, as well as in crop science.

Moreover, pharmaceutical compositions comprising a compound of Formula I, II or III are also disclosed herein. The pharmaceutical composition comprises at least one compound of Formula I, II or III, or a pharmaceutically acceptable salt or solvate of said compound, and at least one pharmaceutically acceptable carrier. For preparing pharmaceutical compositions from the compounds described by this invention, inert, pharmaceutically acceptable carriers can be either solid or liquid. Solid form preparations include powders, tablets, dispersible granules, capsules, cachets and suppositories. The powders and tablets may be comprised of from about 5 to about 95 percent active ingredient. Suitable solid carriers are known in the art, e.g., magnesium carbonate, magnesium stearate, talc, sugar or lactose. Tablets, powders, cachets and capsules can be used as solid dosage forms suitable for oral administration. Liquid form preparations include solutions, suspensions and emulsions. As an example may be mentioned water or water-propylene glycol solutions for parenteral injection or addition of sweeteners and opacifiers for oral solutions, suspensions and emulsions. Liquid form preparations may also include solutions for intranasal administration. Aerosol preparations suitable for inhalation may include solutions and solids in powder form, which may be in combination with a pharmaceutically acceptable carrier, such as an inert compressed gas, e.g. nitrogen. Also included are solid form preparations that are intended to be converted, shortly before use, to liquid form preparations for either oral or parenteral administration. Such liquid forms include solutions, suspensions and emulsions. Preferably the compound to be used according to the invention is administered orally.

In a further aspect, the present invention also relates to a compound, or a dimer or a pharmaceutically acceptable salt or solvate of said compound or dimer, having the general structure shown in Formula I, II or III, as defined above.

The compounds of formula I, II and III described above, i.e. the compounds to be used in accordance with the first, second or third aspect of the invention, can be prepared in high yields using short routes of synthesis.

The invention disclosed herein is exemplified by the following examples which should not be construed to limit the scope of the disclosure. Alternative mechanistic pathways and analogous structures will be apparent to those skilled in the art.

The following solvents and reagents may be referred to by their abbreviations:

DMAP 4-dimethylaminopyridine

| | |
|---|---|
| DMF | dimethylformamide |
| $Et_3N$ or $Net_3$ | triethylamine |
| Log D | distribution coefficient |
| PBS | phosphate buffered saline |
| PAMPA | parallel artificial membrane permeability assay |
| THF | tetrahydrofurane |
| TMEDA | N,N,N',N'-tetramethylethylendiamin |
| TMG | 1,1,3,3-tetramethylguanidin |
| TMSCl | trimethylsilyl chloride |
| TMSOTf | trimethylsilyl triflate |

## Examples

In general, compounds of Formula I can be synthesized in accordance with Scheme 1 (synthesis of benzofuran-2-ylmethanamine building block) and Scheme 2 (synthesis of ureidobenzoic acid building block).

Scheme 1:

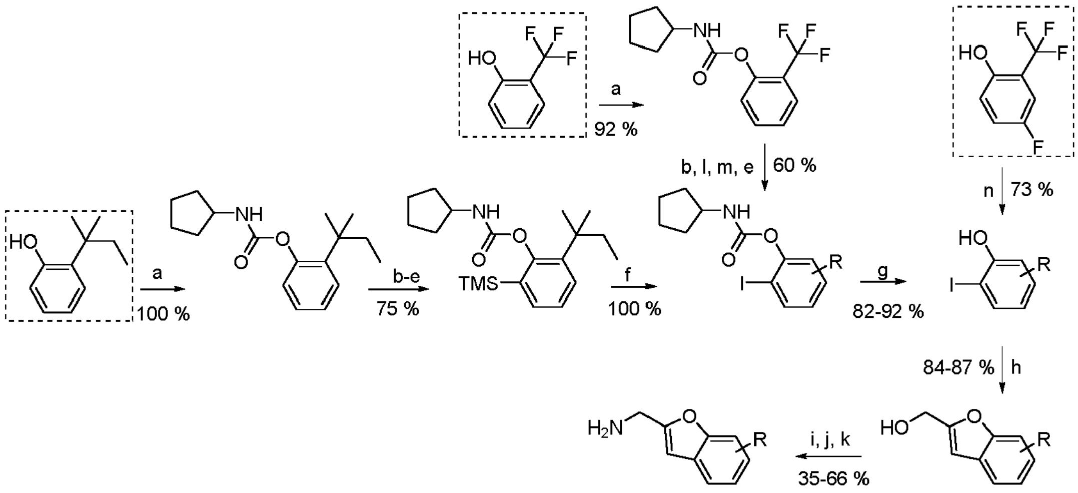


**a:** 0.1 eq. DMAP, 2 eq. cyclopentyl isocyanate, THF, 70 °C, 30 h. **b:** 1.05 eq. TMSOTf, 1.1 eq. TMEDA, $Et_2O$, rt, 1 h. **c:** 1.2 eq. TMSCl, $Et_2O$, -78 °C, 1 h. **d:** 2 eq. TMEDA, 2.2 eq. sec-buli, $Et_2O$, -78 °C, 1 h. **e.** 11 eq. HCl, $MeOH/H_2O/Et_2O$, rt, 2h. **f:** 1.3 eq. ICl, $CH_2Cl_2$, 0°C, rt, 3h. **g.** 1 M NaOH, THF, rt, 4 h. **h:** 3 eq. TMG, 0.1 eq. CuI, 0.05 eq. $PdCl_2(PPh_3)_2$, 1.1 eq prop-2-in-1-ol, DMF, 40 °C, 20 h. **i:** 1.6 eq. thionyl chloride, THF, 50 °C, 90 min. **j** : 3 eq. $NaN_3$, DMF, 50 °C, 15 h. **k.** $H_2$, Pd/C, THF, rt, 20 h. **1:** 2 eq. TMEDA, 2.2 eq. n-buli, $Et_2O$, -78 °C, 90 min. **m:** : 1.25 eq. $I_2$, $Et_2O$, -78 °C, 90 min. **n:** 1.0 eq. ICl, 1.5 eq. $CaCO_3$, MeOH, $H_2O$, rt, 5 h.

Scheme 2:

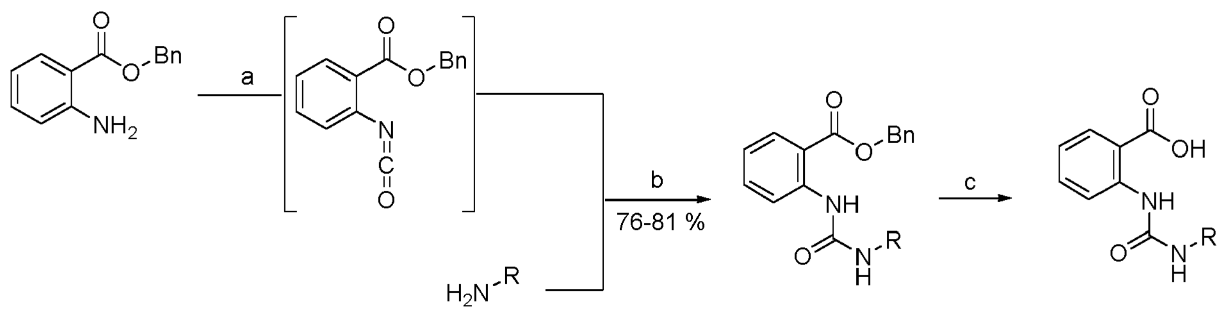


**a.** 0.5 eq. triphosgene, 2.1 eq. triethylamine, THF, 90 min. b: 0.8 eq. amine, THF, 15 h. c. $H_2$, Pd/C, THF, rt, 20 h.

Furthermore, compounds of Formula II can be synthesized in accordance with Scheme 3.

Scheme 3:

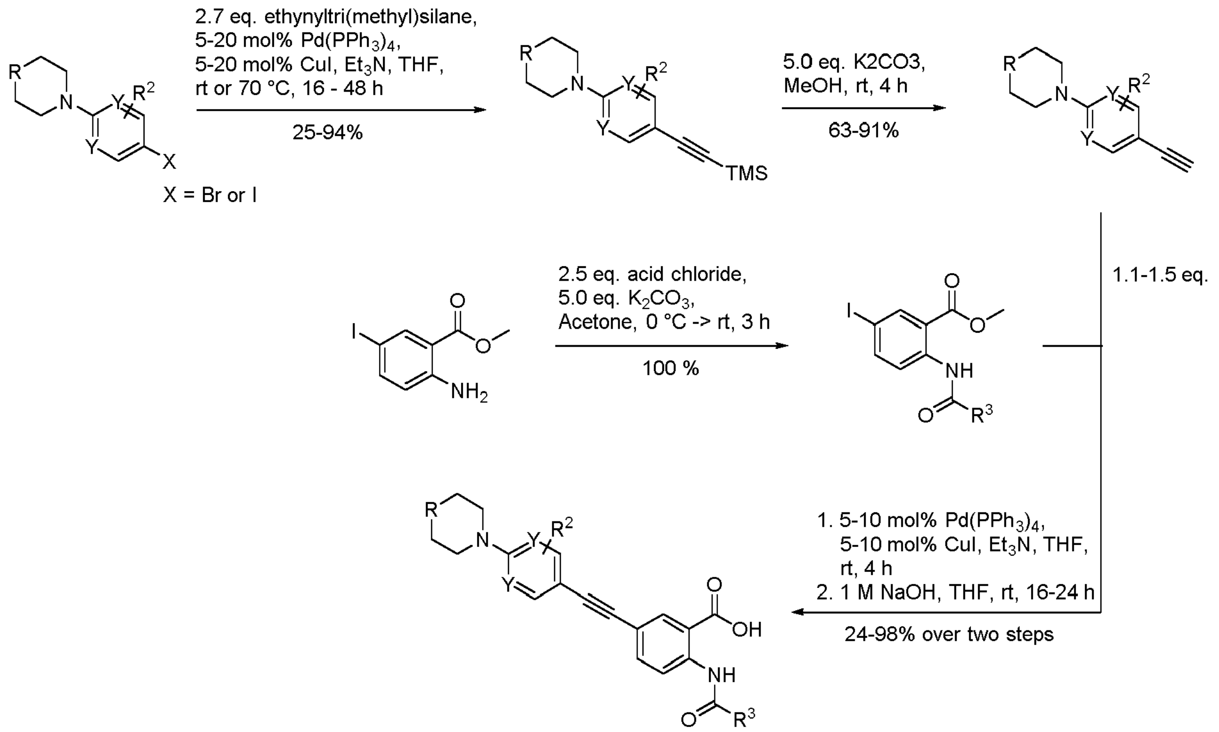


Compounds of Formula II having various $R^8$ substituents can be prepared through the general routes described in Schemes 1 to 14 of WO 2020/225330.

Example 1:

Preparation of

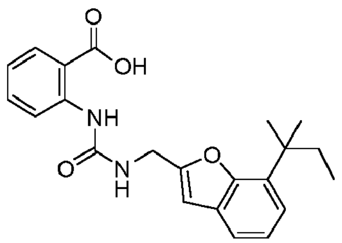

;

The compound of Example 1 was synthesized in accordance with Schemes 1 and 2.

Under nitrogen atmosphere, a 0 °C cooled solution of 2- (2-methylbutan-2-yl) phenol (3.50 mL, 3.43 g, 20.9 mmol, 1 eq.) and 4-(dimethylamino) pyridine (255 mg, 2.09 mmol, 0.1 eq) in abs. tetrahydrofurane (10 mL) was treated with cyclopentyl isocyanate (4.65 mL, 4.58 g, 41.3 mmol, 2 eq.). After stirring at 70 °C for 30 h the mixture was cooled to room temperature, treated with 1 M hydrogen chloride solution (42 mL) and extracted with diethyl ether three times. The combined organic extracts were washed with sat. sodium bicarbonate solution and the separated aqueous phase was reextracted with diethyl ether. Next, the organic phases were combined, dried over sodium sulfate and the solvents were removed in vacuo.
Yield: 5.73 g (20.8 mmol, 100 %) of a white solid.

In an inert atmosphere, a solution of trimethylsilyl triuoromethanesulfonate (4.0 mL, 4.9 g, 22 mmol, 1.05 eq.) in diethyl ether (30 mL) was added dropwise to a solution of 2-(2-methylbutan-2-yl)phenyl cyclopentylcarbamate (5.76 g, 20.9 mmol, 1 eq.) and tetramethylethylenediamine (3.5 mL, 2.7 g, 23 mmol, 1.1 eq.) in diethyl ether (160 mL) . After 1 h stirring at room temperature, the reaction mixture was cooled to -78 °C, treated with trimethylsilyl chloride (3.3 mL, 2.8 g, 26 mmol, 1.2 eq.) and stirred at -78 °C for 1 h. TMEDA (6.3 mL, 4.9 g, 42 mmol, 2.0 eq.) and sec-butyl lithium solution (1.4 M in cyclohexane; 33 mL, 46 mmol, 2.2 eq.) were added successively to the mixture that was afterwards

stirred at -78 °C for 1 h. Finally, the thawing reaction mixture was treated with methanol (2.0 mL) and aqueous hydrogen chloride solution (2M, 125 mL, 0.25 mmol, 11 eq.) and stirred until room temperature was reached. The phases were separated, the aqueous phase was extracted with diethyl ether three times and the combined organic extracts were washed with sat. sodium bicarbonate solution. After reextraction of the separated aqueous phase the combined organic phases were dried over sodium sulfate, concentrated in vacuo and purified by column chromatography (silica gel, petroleum ether/dichloromethane 3:1 v/v).

Yield: 5.43 g (15.6 mmol, 75 %) of a white solid.

Under nitrogen atmosphere, a 0 °C cooled solution of 2-(2-methylbutan-2-yl)-6-(trimethylsilyl)phenyl cyclopentylcarbamate (5.51 g, 15.9 mmol, 1.0 eq.) in abs. dichloromethane (200 mL) was carefully treated with a solution of iodo monochloride (3.35 g, 20.6 mmol, 1.3 eq.) in abs. dichloromethane (20 mL). The reaction mixture was stirred for 3 h while warming up to room temperature and afterwards washed with sat. sodium thiosulfate solution twice. The combined aqueous phases were extracted with dichloromethane three times and the combined extracts were washed with brine. Dehydration over sodium sulfate, concentration in vacuo and purification via column chromatography (silica gel, petroleum ether/$CH_2Cl_2$ 2:1 v/v) finally furnished the pure product.

Yield: 6.38 g (15.9 mmol, 100 %) of a white solid.

2-Iodo-6-(2-Methylbutan-2-yl) cyclopentylcarbamate (6.36 g, 15.9 mmol) was solved in tetrahydrofurane (60 mL) and treated with aqueous sodium hydroxide solution (1M; 48 mL). After vigorous stirring for 4 h the resulting mixture was acidifed to pH 1 by addition of hydrogen chloride solution (1M) and subsequently extracted with dichloromethane three times. The combined organic extracts were dried over sodium sulfate and concentrated under reduced pressure. Finally, the crude product was purified by column chromatography (petroleum ether/$CH_2Cl_2$ 5:1 v/v).

Yield: 4.25 g (14.7 mmol, 92 %) of a colorless liquid.

The reaction was performed under inert conditions. To a solution of 2-iodo-6-(2-methylbutan-2-yl)phenol (1.13 g, 3.88 mmol) in abs. *N*,*N*-dimethylformamide (4 mL) were successively added 1,1,3,3-tetramethylguanidine (1.5 mL, 1.3 g, 12 mmol), bis(triphenylphosphine)-palladium dichloride (146 mg, 194 μmol) and copper (I) iodide (74 mg, 0.39 mmol). After 5 min stirring at room temperature, the reaction mixture was treated dropwise with a solution of 2-propin-1-ol (0.24 mL, 0.23 g, 4.1 mmol) in abs. DMF (8 mL) and then stirred at 40 °C overnight. The resulting mixture was diluted with diethyl ether and washed with water and brine once each. The aqueous phases were reextracted with diethyl ether twice. Finally, after the combined organic phases were dried over sodium sulfate and the solvent was evaporated, the obtained crude product was purified via column chromatography (silica gel, $CH_2Cl_2$).

Yield: 738 mg (3.38 mmol, 87 %) of a yellowish liquid.

Dest. thionyl chloride (70 μL, 0.11 g, 0.96 mmol, 1.6 eq.) was added dropwise to a solution of 7-(2-Methylbutan-2-yl)benzofuran-2-ylmethanol (134 mg, 615 μmol) in abs. tetrahydrofurane (2 mL). The reaction mixture was stirred for 90 min at 50 °C and mixed with dichloromethane, water and brine afterwards. The separated aqueous phase was extracted with dichloromethane three times and all combined organic phases were dehydrated over sodium sulfate. Evaporation of the volatile components and subsequent column chromatography (silica gel, petroleum ether/$CH_2Cl_2$ 5:1 v/v) afforded the product.

Yield: 117 g (493 μmmol, 80 %) of a colorless oil.

Under inert conditions, 2-(Chloromethyl)-7-(2-Methylbutan-2-yl)benzofuran (556 mg,2.34 mmol) was solved in abs. *N*,*N*-dimethylformamide (7 mL) and treated with sodium azide (458 mg, 7.05 mmol) . The suspension was stirred at 50 °C for 11 h, subsequently diluted with water and extracted with diethyl ether twice. The combined organic phases were dried over sodium sulfate and freed from solvent under reduced pressure. The crude product was purified via column chromatography (silica gel, petroleum ether/$CH_2Cl_2$ 10:1 v/v).

Yield: 523 mg (2.15 mmol, 91 %) of colorless crystals.

The obtained 2-(azidomethyl)-7-(2-methylbutan-2-yl)benzofuran (500 mg, 2.05 mmol) was mixed with abs. tetrahydrofurane (20 mL) and palladium on carbon (Pd/C, 10 w%) and stirred under hydrogen atmosphere for 3 h. After dilution with dichloromethane, the catalyst was removed by filtration and the solution was concentrated under reduced pressure. The obtained crude product was purifed via column chromatography (silica gel, $CH_2Cl_2/CH_3OH$ gradient 99:1 to 19:1 v/v).

Yield: 278 mg (1.28 mmol, 62 %) of a colorless liquid.

Under nitrogen atmosphere, benzyl anthranilate (363 mg, 1.60 mmol) was solved in abs. tetrahydrofurane (12 mL) and added slowly to a solution of triphosgene (0.24 g, 0.80 mmol) in abs. tetrahydrofurane (1 mL). After careful, dropwise addition of abs. triethylamine (0.44 mL, 0.32 g, 3.2 mmol), the milky reaction mixture was stirred for 90 min at room temperature. Subsequently, excess phosgene was removed by degassing and volatile reaction components were evaporated. The solid residue was suspended in abs. tetrahydrofurane (13 mL) and treated with 7-(2-Methylbutan-2-yl)benzofuran-2-yl)methanamine (278 mg, 1.28 mmol). The reaction mixture was stirred overnight, diluted with water and extracted with dichloromethane three times. Dehydration of the combined organic phases over sodium sulfate and subsequent evaporation of the solvents afforded the crude product that was finally purified via column chromatography (silica gel, petroleum ether/$CH_2Cl_2$ 1:3 v/v).

Yield: 485 mg (1.03 mmol, 81 %) of a colorless solid.

Benzyl 2-(3-((7-(2-methylbutan-2-yl)benzofuran-2-yl)methyl)ureido) benzoate (453 mg, 0.96 mmol) was mixed with abs. tetrahydrofurane (10 mL) and palladium on carbon (45 mg) and stirred under hydrogen atmosphere for 19 h. After dilution with dichloromethane, the catalyst was removed by filtration and the solution was concentrated under reduced pressure. The obtained crude product was purified via multiple purification steps using column chromatography (silica gel, petroleum ether/$CH_2Cl_2$/$CH_3COOH$ 16:4:1 v/v and $CH_2Cl_2$/$CH_3OH$ 50:1 v/v).

Yield: 52.0 mg (0.137 mmol, 13 %) of a white solid.

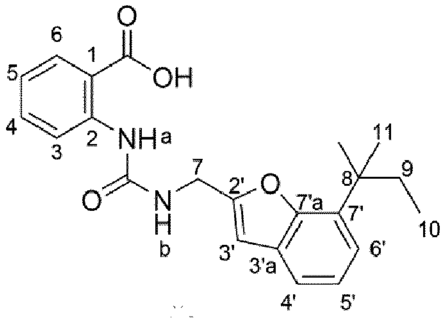

$^1H$ NMR (500 MHz, DMSO-$d_6$) : δ [ppm] = 13.30 (s, 1H, COOH) 10.30 (s, 1H, NH-a), 8.38 (dd, $^3J_{HH}$ = 8.4 Hz, $^4J_{HH}$ = 0.5 Hz, 1H, H-3), 8.12-8.00 (m, 1H, NH-b), 7.91 (dd, $^3J$ HH = 7.9 Hz, $^4J_{HH}$ = 1.7 Hz, 1H, H-6), 7.48 (ddd, $^3J_{HH}$ = 8.7 Hz, 7.3 Hz, $^4J_{HH}$ = 1.6 Hz, 1H, H-4), 7.41 (dd, $^3J_{HH}$ = 7.6 Hz, $^4J_{HH}$ = 1.1 Hz, 1H, H-4'), 7.13 (t, $^3J_{HH}$ = 7.6 Hz, 1H, H-5'), 7.06 (dd, $^3J_{HH}$ = 7.6 Hz, $^4J_{HH}$ = 1.1 Hz, 1H, H-6'), 7.00-6.94 (m, 1H, H-5), 6.67 (s, 1H, H-3'), 4.45 (d, $^3J_{HH}$ = 5.7 Hz, 2H, H-7), 1.88 (q, $^3J_{HH}$ = 7.4 Hz, 2H, H-9), 1.39 (s, 6H, H-11), 0.57 (t, $^3J_{HH}$ = 7.4 Hz, 3H, H-10).

$^{13}C$ NMR (125 MHz, DMSO-$d_6$) : δ [ppm] = 169.5 (COOH), 155.4 (CONH), 154.7 (C-2'), 152.2 (C-7'a), 142.9 (C-2), 140.6 (C-1"), 133.6 (C-4), 132.2 (C-7'), 130.8 (C-6), 128.6 (C-3'a), 122.6 (C-5'), 121.4 (C-6'), 120.2 (C-5), 119.2 (C-3), 118.8 (C-4'), 114.8 (C-1), 102.7 (C-3'), 37.4 (C-7), 36.9 (C-8), 33.6 (C-9), 27.2 (C-11), 9.3 (C-10).

ATR-IR: $\tilde{v}$ [$cm^{-1}$] = 3230, 2962, 2875, 1683, 1659, 1606, 1586, 1557, 1466, 1452, 1413, 1384, 1321, 1266, 1237, 1161, 1088, 1044, 949, 922, 859, 807, 745, 717, 696, 657, 630, 612, 565, 524, 482, 452, 425, 406.

HRMS ($ESI^+$): m/z = 379.1665 (379.1663 calculated for $[M-H]^-$).

Mp= 138.3 °C

Example 2:

Preparation of

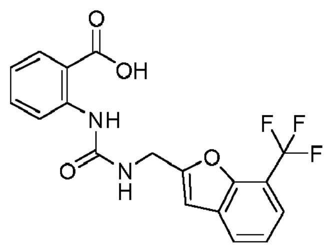

:

The compound of Example 2 was synthesized using the procedure of Schemes 1 and 2.

2-(Trifluoromethyl)phenyl cyclopentylcarbamate was synthesized via the procedure described in Example 1, using 2-(triuoromethyl)phenol (1.19 mL, 1.62 g, 9.98 mmol) as phenol derivative and 1.24 mL (1.22 g, 11.0 mmol) cyclopentyl isocyanate.

Yield: 2.50 g (9.16 mmol, 92 %) of a colorless solid.

In an inert atmosphere, a solution of trimethylsilyl triuoromethanesulfonate (0.25 mL, 0.31 g, 1.4 mmol, 1.05 eq.) in diethyl ether (2 mL) was added dropwise to a solution of 2-(trifluoromethyl)phenyl cyclopentylcarbamate (360 mg, 1.32 mmol, 1 eq.) and tetramethylethylenediamine (TMEDA, 0.22 mL, 0.17 g, 1.4 mmol, 1.1 eq.) in diethyl ether (8 mL). After 1 h stirring at room temperature, the reaction mixture was cooled to -78 °C and treated with TMEDA (0.40 mL, 0.31 g, 2.6 mmol, 2 eq.) and n-butyl lithium solution (1.4 M in cyclohexane; 1.85 mL, 2.6 mmol, 2 eq.) successively. At -78 °C, the mixture was stirred for 90 min, treated with a solution of iodine (0.42 g, 1.6 mmol, 1.25 eq.) in diethyl ether (1 mL) and again stirred for 90 min. Finally, the thawing reaction mixture was treated with methanol (0.2 mL) and aqueous

hydrogen chloride solution (2M, 8 mL, 16 mmol, 12 eq.) and stirred until room temperature was reached. The phases were separated, the aqueous phase was extracted with diethyl ether three times and the combined organic extracts were washed with sat. sodium bicarbonate solution. After reextraction of the separated aqueous phase the combined organic phases were dried over sodium sulfate, concentrated in vacuo and puri_ed by column chromatography (silica gel, petroleum ether/ethyl acetate gradient 50:1 to 10:1 v/v) .

Yield: 315 mg (789 μmol, 60 %) of a colorless solid.

2-Iodo-6-(trifluoromethyl) cyclopentylcarbamate was converted to the target compound 2-(3-((7-(trifluoromethyl)benzofuran-2-yl)methyl)ureido)benzoic acid in seven steps, analogous to Example 1.

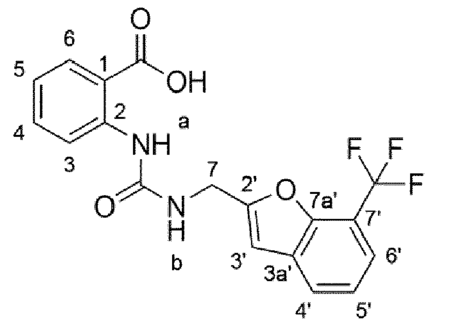

$^1$H NMR (500 MHz, DMSO-$d_6$): δ [ppm] = 13.50 (brs, 1H, COOH) 10.77 (brs, 1H, NH-a), 8.36 (dd, $^3J_{HH}$ = 8.5 Hz, $^4J_{HH}$ = 1.1 Hz, 1H, H-3), 8.10 (brs, 1H, NH-b), 7.95-7.89 (m, 2H, H-6, H-4'), 7.60 (d, 3J HH = 7.6 Hz, 1H, H-6'), 7.46-7.38 (m, 2H, H-4, H-5'), 7.97-6.92 (m, 1H, H-5), 6.91 (s, 1H, H-3'), 4.49 (d, $^3J_{HH}$= 5.6 Hz, 2H, H-7).

$^{13}$C NMR (125 MHz, DMSO-$d_6$): δ [ppm] = 169.8 (COOH), 158.4 (C-2'), 154.8 (CONH), 149.5 (C-7'a), 142.7 (C-2), 133.0 (C-4), 131.0 (C-6), 130.0 (C-3'a), 125.8 (C-4'), 123.4 (q, 1J CF = 272.1 Hz, CF3)) 123.0 (C-5'), 120.8 (q, $^3J_{CF}$ = 4.4 Hz, C-6') 120.1 (C-5), 118.9 (C-3), 116.5 (C-1), 112.7 (q, $^2J_{CF}$ = 33.6 Hz, C-7'), 103.4 (C-3'), 36.8 (C-7).

$^{19}$F NMR (565 MHz, DMSO-$d_6$) : δ [ppm] = -59.47 ($CF_3$).

ATR-IR: $\tilde{v}$ [cm$^{-1}$] = 3330, 2928, 2883, 2652, 2559, 1687, 1651, 1561, 1430, 1412, 1325, 1293, 1267, 1241, 1171, 1131, 1118, 1085, 1051, 945, 817, 755, 740, 733, 707, 667, 612, 597, 560, 522, 482, 456, 418

HRMS (ESI$^-$): m/z = 377.0749 (377.0755 calculated for [M-H]$^-$).

Mp = 179.5 °C

Example 3:

Preparation of:

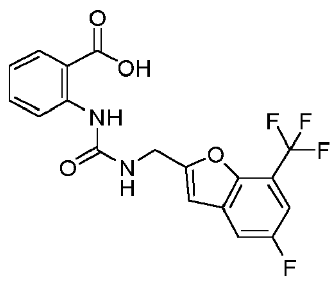

The compound of Example 3 was synthesized using the procedure of Schemes 1 and 2.

4-Fluoro-2-(trifluoromethyl)phenol was synthesized by treating a solution of 1.80 g (10.0 mmol, 1.0 equiv.) 4-fluoro-2-(trifluoromethyl)phenol in 5 mL methanol and with a suspension of 1.50 g (15.0 mmol, 1.5 equiv.) calcium carbonate in 6 mL water and a solution of 1.62 g (10.0 mmol, 1.0 equiv.) iodine monochloride in 4 mL methanol, successively. The reaction mixture was stirred at room temperature for 5 h before it was diluted with water and dichloromethane. After phase separation, the aqueous phase was extracted three times with dichloromethane. The combined organic phases were washed with brine and saturated sodium thiosulphate solution, once each. The organic phase was dried over sodium sulphate and the solvent was removed under reduced pressure. Purifcation of the crude product was performed by column chromatography (silica gel, PE/CH2C12 4:1 v/v).

Yield: 2.23 g (7.28 mmol, 73 %) of a yellow oil.

4-Fluoro-2-iodo-6-(trifluoromethyl)phenol was converted to the target compound 2-(3-((7-(trifluoromethyl)benzofuran-2-yl)methyl)ureido)benzoic acid in six steps, analogous to Example 1 and 2.

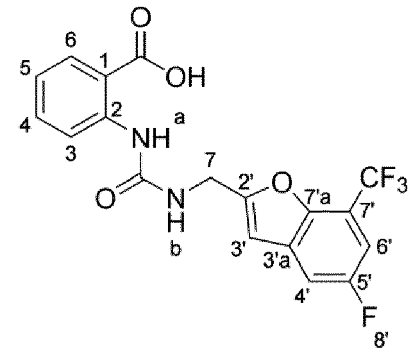

$^1$H NMR (600 MHz, DMSO-$d_6$) : δ [ppm] = 13.35 (brs, 1H, COOH) 10.30 (brs, 1H, NH-a), 8.38 (dd, $^3J_{HH}$ = 8.6 Hz, $^4J_{HH}$ = 1.2 Hz, 1H, H-3), 8.21 (t, $^3J_{HH}$ = 5.7 Hz, 1H, NH-b), 7.92 (dd, $^3J_{HH}$ = 8.6 Hz, $^4J_{HH}$ = 1.2 Hz, 1H, H-6), 7.79 (dd, $^3J_{HH}$ = 8.5 Hz, $^4J_{HH}$ = 2.6 Hz, 1H, H-4'), 7.56 (dd, $^3J_{HH}$ = 9.2 Hz, $^4J_{HH}$ = 2.6 Hz, 1H, H-6'), 7.49 (ddd, $^3J_{HH}$ = 8.7 Hz, $^3J_{HH}$ = 7.2 Hz, $^4J_{HH}$ = 1.8 Hz, 1H, H-4), 7.98 (ddd, $^3J_{HH}$ = 8.1 Hz, $^3J_{HH}$ = 7.2 Hz, $^4J_{HH}$ = 1.2 Hz, 1H, H-5), 6.92 (s, 1H, H-3'), 4.49 (d, $^3J_{HH}$ = 5.5 Hz, 2H, H-7).

$^{13}$C NMR (150 MHz, DMSO-$d_6$) : δ [ppm] = 169.5 (COOH), 160.4 (C-2'), 157.5 (d, $^1J_{CF}$ = 238.3 Hz, C-5'), 154.6 (CONH), 145.9 (C-7'a), 142.8 (C-2), 134.2 (C-4), 131.5 (d, $^3J_{CF}$ = 10.9 Hz, 3'a), 131.0 (C-6)$_f$ 122.5 (d, $^1J_{CF}$ = 269.9 Hz, $CF_3$), 120.4 (C-5), 119.2 (C-3), 114.7 (C-1), 111.6 (d, $^2J_{CF}$ = 25.0 Hz, C-4'), 108.9 (dq, $^2J_{CF}$ = 29.7 Hz, $^3J_{CF}$ = 4.9 Hz, C-6'), 103.9 (d, $^4J_{CF}$ = 4.9 Hz, C-3'), 36.8 (C-7).

$^{19}$F NMR (565 MHz, DMSO-$d_6$) : _[ppm] = -59.75 ($CF_3$), -118.86 (C-5'-F).

ATR-IR: $\tilde{v}$ [cm$^{-1}$] = 3333, 2913, 2648, 1686, 1655, 1607, 1586, 1561, 1474, 1437, 1428, 1410, 1370, 1328, 1318, 1267, 1183, 1162, 1130, 1114, 1085, 997, 953, 917, 866, 827, 762, 754, 729, 705, 681, 665, 629, 586, 559, 519, 492, 442, 415.

HRMS (ESI$^+$): m/z = 395.0467 (395.0660 calculated for [M-H]$^-$).

Example 4:

Preparation of:

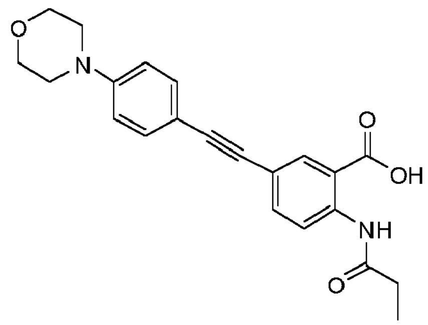

The compound of Example 4 was synthesized in accordance with Scheme 3.

A mixture of 4-(4-iodophenyl)morpholine (0.78 mL, 3.46 mmol, 1.0 eq.), Copper iodide (32.9 mg, 0.173 mmol, 5 mol%), abs. tetrahydrofuran (6 mL) and triethylamine (17 mL) was degassed with nitrogen for 30 minutes. Then, $Pd(PPh_3)_4$ (200 mg, 0.173 mmol, 5 mol%) and ethynyltri(methyl)silane (1.29 mL, 9.34 mmol, 2.7 eq.) were added to the mixture. After stirring at room temperature for 20 h the reaction was quenched by the addition of water and extracted with ethyl acetate three times. The combined organic layer was dried over sodium sulfate, filtered and concentrated in vacuo. The crude product was purified via column chromatography (dichloromethane + 0.5% v/v acetone).
Yield: 678 mg (2.61 mmol, 76%) of an orange solid.

Under nitrogen atmosphere, 4-(4-((trimethylsilyl)ethynyl)phenyl)morpholine (372 mg, 1.43 mmol, 1.0 eq.) was dissolved in methanol (12 mL) and potassium carbonate (990 mg, 7.16 mmol, 5.0 eq.) was added. After stirring for 4 h at room temperature the mixture was filtered and evaporated under low pressure. The crude product was re-dissolved in dichloromethane and washed with water. Then, the organic layer was dried over sodium sulfate, filtered and concentrated under vacuo. The crude product was purified by column chromatography (dichloromethane + 1% v/v acetone). Yield: 205 mg (1.10 mmol, 77%) of an orange solid.

Copper iodide (5.7 mg, 0.030 mmol, 5 mol%) was added to a solution of methyl-5-iodo-2-propionamidoanthranilate (200 mg, 0.600 mmol, 1.0 eq.) in abs. tetrahydrofuran (3 mL) and triethylamine (4 mL). After the mixture was degassed with nitrogen for 30 minutes $Pd(PPh_3)_4$ (34.7 mg, 0.030 mmol, 5 mol%) and 4-(4-ethynylphenyl)morpholine (169 mg, 0.901 mmol, 1.5 eq.) were added. The resulting mixture was stirred for 4 h at room temperature and then quenched by the addition of water. The aqueous phase was extracted with dichloromethane three times and the combined organic layer was dried over sodium sulfate, filtered and concentrated under reduced pressure. The crude product was purified via column chromatography (petroleum ether/ethyl acetate 2:1 v/v).
Yield: 156 mg (0.397 mmol, 66%) of a yellow solid.

Methyl 5-((4-cyclohexylphenyl)ethynyl)-2-propionamidobenzoate (149 mg, 0.378 mmol, 1.0 eq.) was dissolved in tetrahydrofuran (5 mL) and sodium hydroxide solution (1 M, 2 mL) was added. After stirring for 18 h tetrahydrofuran was removed under reduced pressure and the product was precipitated by the addition of hydrogen chloride solution (1 M, 3 mL). The solid was filtered and washed with water.
Yield: 137 mg (0.362 mmol, 96%) of a slightly yellow solid.

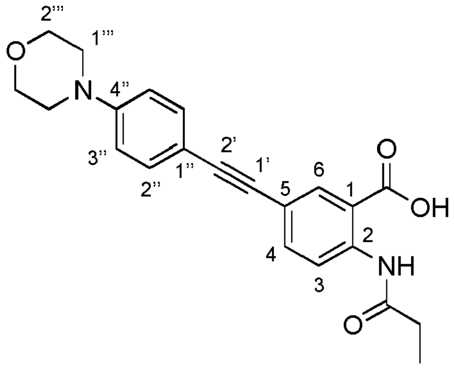

$^1$H-NMR (400 MHz, DMSO-$d_6$) : $\delta$ [ppm] = 11.30 (s, 1H, N*H*CO), 8.55 (d, $^3J_{H,H}$ = 8.8 Hz, 1H, H-3), 8.05 (d, $^2J_{H,H}$ = 2.1 Hz, 1H, *H*-6), 7.67 (dd, $^3J_{H,H}$ = 8.7 Hz, $^2J_{H,H}$ = 2.2 Hz, 1H, *H*-4), 7.40 (d, $^3J_{H,H}$ = 8.8 Hz, 2H, *H*-2"), 6.95 (d, $^3J_{H,H}$ = 9.0 Hz, 2H, *H*-3"), 3.78-3.69 (m, 4H, *H*-2'''), 3.21-3.14 (m, 4H, *H*-1'''), 2.42 (q, $^3J_{H,H}$ = 7.5 Hz, 2H, C*H*$_2$), 1.13 (t, $^3J_{H,H}$ = 7.5 Hz, 3H, C*H*$_3$).

$^{13}$C-NMR (101 MHz, DMSO-$d_6$) : $\delta$ [ppm] = 172.1 (NHCO), 168.9 (COOH), 150.8 (*C*-4"), 140.5 (*C*-2), 136.0 (*C*-4), 133.6 (*C*-6), 132.4 (*C*-2"), 119.9 (*C*-3), 116.8 (*C*-5), 116.8 (*C*-1), 114.4 (*C*-3"), 111.5 (*C*-1"), 90.0 (*C*-2'), 86.7 (*C*-1'), 65.9 (*C*-2'''), 47.4 (*C*-1'''), 30.7 (*C*H$_2$), 9.29 (*C*H$_3$).

ATR-IR: $\tilde{v}$ [cm$^{-1}$] = 2972, 2923, 2835, 2613, 1686, 1584, 1518, 1380, 1288, 1228, 1182, 1107, 926, 791, 729.

HRMS (ESI$^+$): m/z = 379.1691 [M+H]$^+$ (calculated: 379.1653 [M+H]$^+$).

Examples 5-16:

Furthermore, using the general procedure of Scheme 3, the following compounds of Formula II were synthesized:

| Example | Compound |
|---|---|
| 5 | 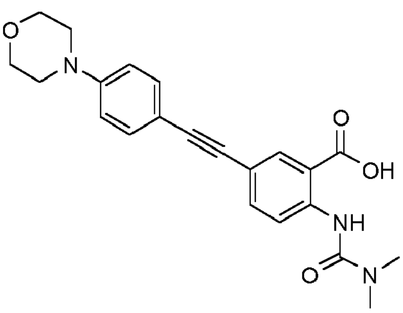 |

(continued)

| Example | Compound |
|---|---|
| 6 | 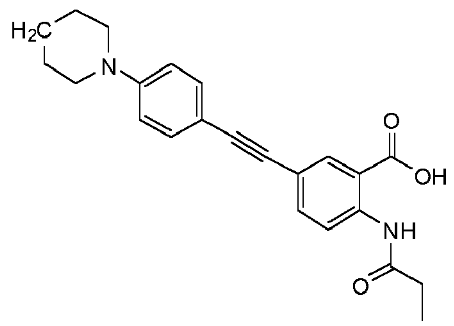 |
| 7 | 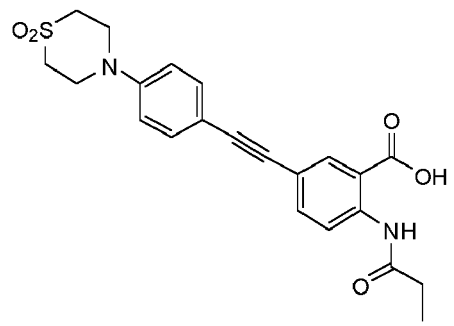 |
| 8 | 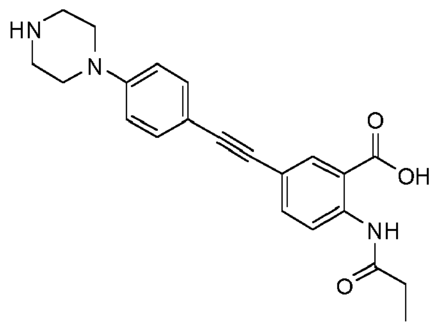 |
| 9 | 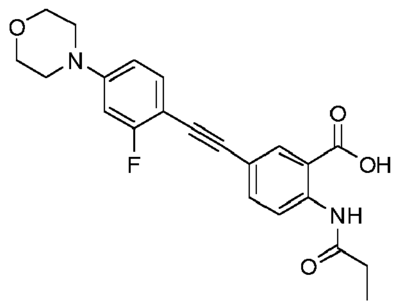 |

(continued)

| Example | Compound |
|---|---|
| 10 | 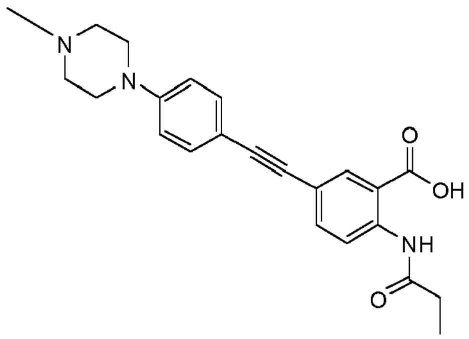 |
| 11 | 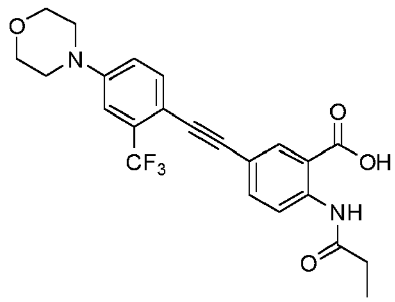 |
| 12 | 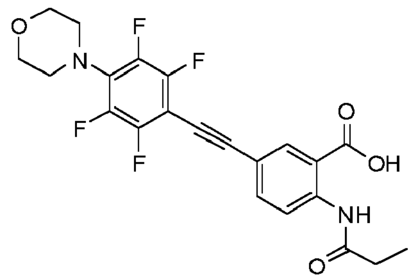 |
| 13 | 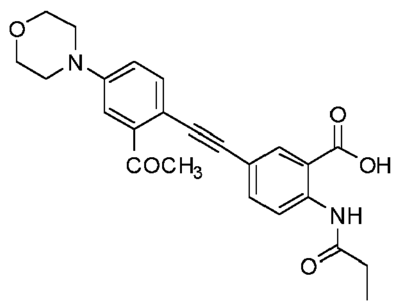 |
| 14 | 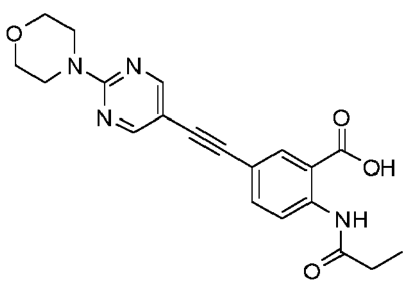 |

(continued)

| Example | Compound |
|---|---|
| 15 | 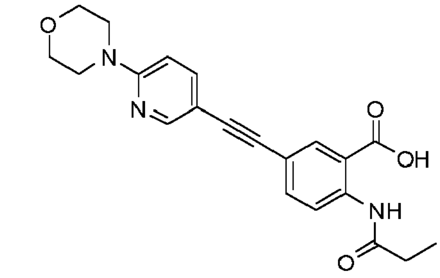 |
| 16 | 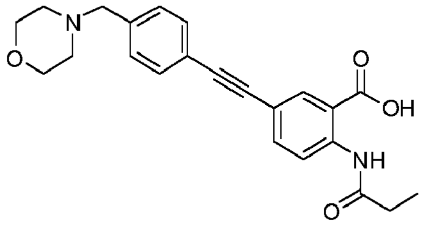 |

In cases where the aryl halides were not commercially available they were synthesized using copper catalyzed Ullman-Goldberg coupling reaction according to the following scheme.

Scheme 4:

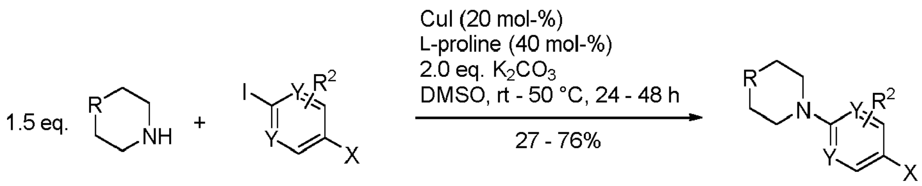


X = Br or I

Further aryl iodides were synthesized via nucleophilic aromatic substitution (Scheme 5) or boric acid/glycerol catalyzed double michael addition reaction, respectively (Scheme 6).

Scheme 5:

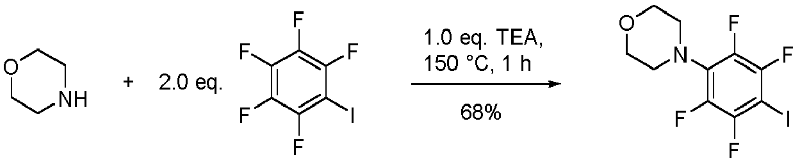


Scheme 6:

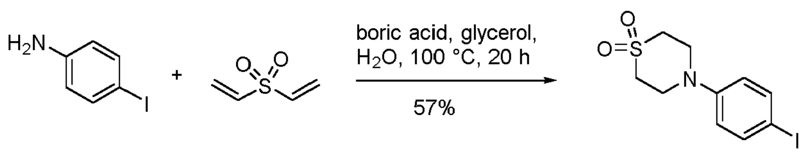


4-Morpholinobenzene ethynyl anthranilic acid bearing an urea function was synthesized using 4-nitrophenyl

chloroformate and dimethylamine followed by palladium and copper catalyzed sonogashira cross coupling reaction according to Scheme 7.

Scheme 7:

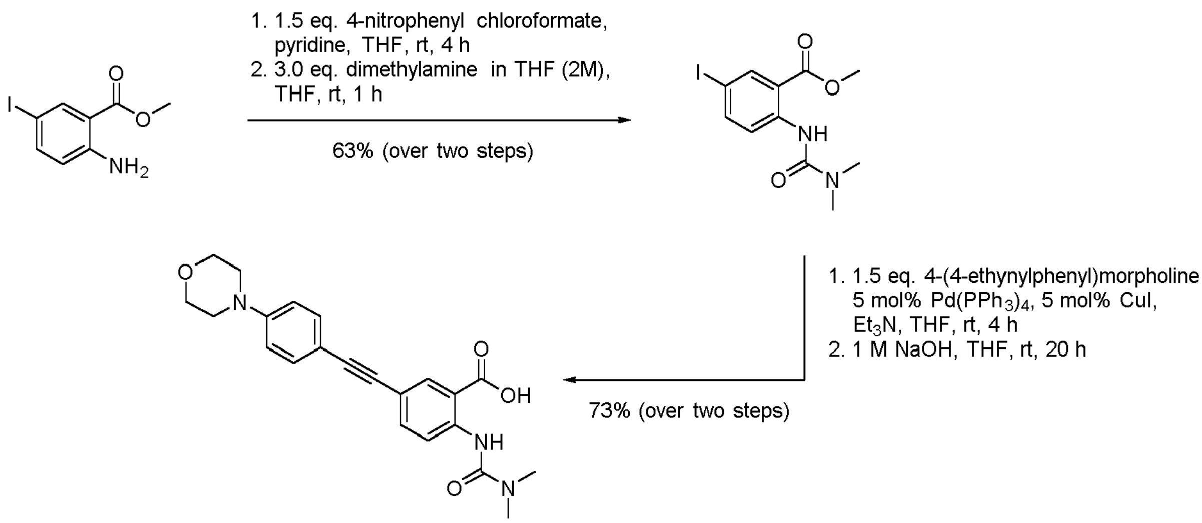


Comparative Example 17:

Preparation of

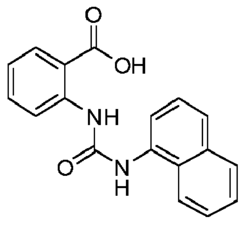

:

The compound of Example 17 was synthesized in accordance with Scheme 2.

Under nitrogen atmosphere, benzyl anthranilate (341 mg, 1.50 mmol) was solved in abs. tetrahydrofurane (10 mL) and added slowly to a solution of triphosgene (156 mg, 525 µmol) in abs. tetrahydrofurane (1 mL). After careful, dropwise addition of abs. triethylamine (0.42 mL, 0.30 g, 3.0 mmol), the milky reaction mixture was stirred for 90 min at room temperature. Subsequently, excess phosgene was removed by degassing and volatile reaction components were evaporated. The solid residue was suspended in abs. tetrahydrofurane (10 mL/mmol) and treated with naphthyl-1-amine (193 mg, 1.35 mmol). The reaction mixture was stirred overnight, diluted with water and extracted with dichloromethane three times. Dehydration of the combined organic phases over sodium sulfate and subsequent evaporation of the solvents afforded the crude product that was finally purfied via column chromatography (silica gel, $CH_2Cl_2$).
Yield: 409 mg (1.03 mmol, 76 %) of a colorless solid.

Benzyl 2-(3-(naphthalen-1-yl)ureido)benzoate (371 mg, 937 µmol) was mixed with abs. tetrahydrofurane (10 mL) and palladium on carbon (37 mg) and stirred under hydrogen atmosphere for 19 h. After dilution with dichloromethane, the catalyst was removed by filtration and the solution was concentrated under reduced pressure. The obtained crude product was purified via multiple purification steps using column chromatography (silica gel, $CH_2Cl_2/CH_3OH$ gradient 30:1 to 10:1 v/v).
Yield: 186 g (606 µmol, 65 %) of a white solid.

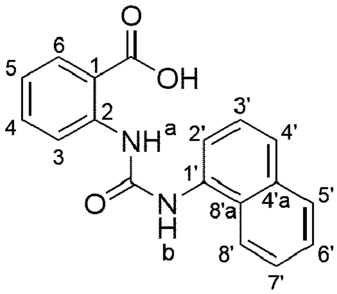

$^1H$ NMR (400 MHz, DMSO-$d_6$) : δ [ppm] = 11.18 (brs, 1H, NH-a), 9.55 (s, 1H, NH-b), 8.34 (dd, $^3J_{HH}$ = 8.5 Hz, $^4J_{HH}$ = 1.1 Hz, 1H, H-3), 8.20-8.09 (m, 1H, H-8'), 8.00-7.87 (m, 2H, H-6, H-5'), 7.73 (d, $^3J_{HH}$ = 8.2 Hz, 1H, H-4'), 7.69 (d, $^3J_{HH}$ = 7.4 Hz, 1H, H-2'), 7.58-7.50 (m, 2H, H-6', H-7'), 7.49 (t, $^3J_{HH}$ = 8.2 Hz, 1H, H-3'), 7.44 (ddd, $^3J_{HH}$ = 8.6 Hz, $^3J_{HH}$ = 7.1 Hz, $^4J_{HH}$ = 1.7 Hz, 1H, H-4), 6.97 (t, $^3J_{HH}$ = 7.6 Hz, 1H, H-5).

$^{13}C$ NMR (125 MHz, DMSO-$d_6$) : δ [ppm] = 169.8 (COOH), 153.6 (CONH), 142.1 (C-2), 134.2 (C-8'a), 133.8 (C-4'a), 132.5 (C-4), 131.0 (C-6), 128.3 (C- 1'), 128.1 (C-5'), 125.9-125.6 (C-3', C-6', C-7'), 124.6 (C-4'), 122.9 (C-8'), 121.5 (C-2'), 120.5 (C-5), 119.5 (C-3), 117.1 (C-1).

ATR-IR: $\tilde{\nu}$ [cm$^{-1}$] = 3186, 3056, 1666, 1583, 1505, 1474, 1447, 1391, 1342, 1318, 1290, 1271, 1251, 1162, 865, 780, 752, 657, 618, 542, 524, 493, 449, 418.

HRMS (ESI+): m/z = 345.0616 (345.0636 calculated for $[M+K]^+$).

Example 18:

Preparation of

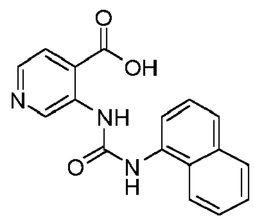

The compound of Example 18 was synthesized in accordance with Scheme 8.

Scheme 8:

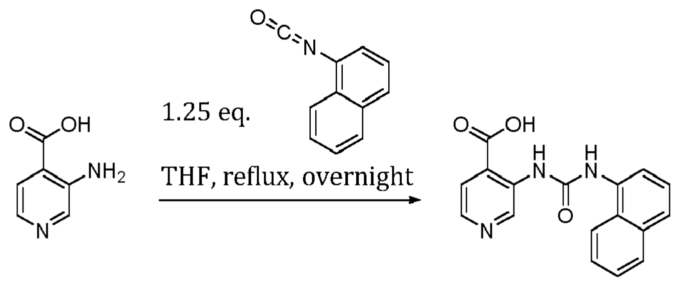


3-Aminoisonicotinic acid (553 mg, 4.00 mmol, 1 eq.) was suspended in abs. tetrahydrofurane (2 mL) and 1-naphthyl isocyanate (1.2 mL, 0.85 g, 5.0 mmol, 1.25 eq.) was added dropwise. The reaction mixture was stirred under reflux for 16 hours and treated with a small amount of methanol, subsequently. After evaporating the solvent, the crude product was purified by column chromatography (silica gel, $CH_2Cl_2/CH_3OH/CH_3COOH$ 100:10:3 v/v).

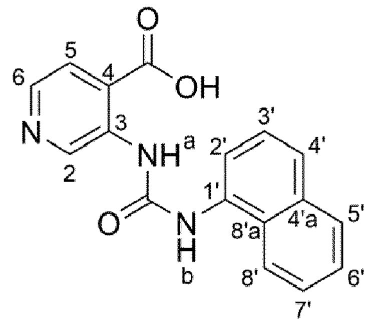

$^1H$ NMR (600 MHz, DMSO-$d_6$) : δ [ppm] = 12.46 (brs, 1H, NH-a), 9.51 (s, 1H, NH-b), 9.42 (s, 1H, H-2) 8.17-8.12 (m, 1H, H-8'), 8.09 (d, $^3J_{HH}$ = 4.9 Hz, 1H, H-6), 8.94-7.89 (m, 1H, H-5'), 7.74 (d, $^3J_{HH}$ = 4.9 Hz, 1H, H-5), 7.71 (d, $^3J_{HH}$ = 8.2 Hz, 1H, H-4'), 7.65 (d, $^3J_{HH}$ = 7.4 Hz, 1H, H-2'), 7.55-7.50 (m, 2H, H-6', H-7'), 7.48 (t, $^3J_{HH}$ = 7.8 Hz, 1H, H-3').

$^{13}C$ NMR (151 MHz, DMSO-$d_6$) : δ [ppm] = 167.9 (COOH), 153.8 (CONH), 141.4 (C-2), 141.1 (C-6), 137.4 (C-3), 134.6 (C-8'a), 133.8 (C-4'a), 130.5 (C-4), 128.4 (C-1'), 128.0 (C-5'), 125.8 (C-6'), 125.7 (C-3'), 125.4 (C-7'), 124.4 (C-4'), 124.0 (C-5), 123.2 (C-8'), 121.6 (C-2').

ATR-IR: $\tilde{\nu}$ [$cm^{-1}$] = 3264, 2921, 2851, 1665, 1630, 1582, 1547, 1504, 1428, 1380, 1345, 1309, 1275, 1258,1238, 792, 770, 741, 711, 674.

HRMS ($ESI^+$): m/z = 308.1040 (308.1030 calculated for $[M+H]^+$).

Example 19: Antiviral Assay

Viruses

CCHFV strain Afg-09 2990 (Ölschläger et al., Complete sequence and phylogenetic characterisation of Crimean-Congo hemorrhagic fever virus from Afghanistan. J. Clin. Virol. 2011, 50, 90-92) had been isolated at BNITM laboratory and passaged ≤3 times before it has been used in this study. LASV strain Ba366 (Lecompte et al., J. Mastomys natalensis and Lassa fever, West Africa. Emerg. Infect. Dis. 2006, 12, 1971-1974) has beenproduced recombinantly and has been passaged at BNITM laboratory ≤3 times. EBOV strain Mayinga 1976 has been provided around 1980 by the Center for Disease Control, Atlanta, Georgia. The passage history since 1976 is not documented. All virus stocks have been grown on Vero 81 cells, quantified by immunofocus assay (see below) and stored at -80°C until use.

Antiviral and toxicity testing *in vitro*

All test compounds were dissolved in DMSO at a concentration of 100 mM and stored at 20 °C. Vero 81 cells were grown in Dulbecco's Modified Eagle's Medium (DMEM) supplemented with 3 % fetal calf serum (FCS) and streptomycin/penicillin and seeded at a density of 1 x 104 cells per well of a 96-well plate one day before infection. Cells were inoculated with LASV Ba366, CCHFV Afg 09-2990 or EBOV Mayinga at a multiplicity of infection (MOI) of 0.01 in the BSL-4 laboratory. The inoculum was removed after 1 h and replaced by fresh medium complemented with different concentrations of compound. Concentration of infectious virus particles in cell culture supernatant was measured three days (LASV and CCHFV) or four days (EBOV) post infection (p.i.) by immunofocus assay. Cell growth and viability under compound treatment was determined by the 3-(4,5-dimethylthiazol-2-yl)-2,5-diphenyl-2H-tetrazoliumbromide (MTT) method as described previously (Günther et el., Application of real-time PCR for testing antiviral compounds against Lassa virus, SARS coronavirus and Ebola virus in vitro. Antiviral Res. 2004, 63, 209-215). A sigmoidal dose-response curve was fitted to the data using Prism GraphPad 6.0 (GraphPad Software). The inhibitory concentrations that reduced the virus titer by 50 % (IC50) were calculated from the sigmoidal functions.

Virus titration

Infectious virus particles were determined by immunofocus assay as previously described (Günther et el., Application of real-time PCR for testing antiviral compounds against Lassa virus, SARS coronavirus and Ebola virus in vitro. Antiviral Res. 2004, 63, 209-215). Briefly, 1x106 Vero cells per 96-well plates were inoculated with 50 μl of serial 10-fold dilutions of sample. The inoculum was removed after 1 h and replaced by a 1 %-methylcellulose - medium overlay. After three days of incubation, cells were fixed with 4 % formaldehyde, washed with phosphate-buffered saline (PBS), and permeabilized with 0.5 % Triton X-100 in PBS. After washing with PBS and blocking with 2 % FCS in PBS, cell foci infected with EBOV, CCHFV or LASV were detected with nucleoprotein (NP)-specific monoclonal antibodies

(LASV and CCHFV) or polyclonal antiserum (EBOV) against the whole virus. After washing, cells were incubated with peroxidase-labeled anti-mouse IgG. Foci were visualized with tetramethylbenzidine and counted.

The antiviral activity obtained with compounds of the examples against several RNA virus families is shown in the table below.

| Virus | Parameter [μM] | Example | | |
|---|---|---|---|---|
| | | 1 | 2 | 4 |
| LASV | $IC_{50}$ | 0.035 | 1.00 | 0.60 |
| | $CC_{50}$ | > 1.0 | n.d.[a] | n.d.[a] |
| EBOV | $IC_{50}$ | 0.014 | n.d.[a] | n.d.[a] |
| | $CC_{50}$ | > 1.0 | n.d.[a] | n.d.[a] |
| CCHFV | $IC_{50}$ | 0.10 | n.d.[a] | n.d.[a] |
| | $CC_{50}$ | > 1.0 | n.d.[a] | n.d.[a] |
| [a]not determined | | | | |

Example 20: hDHODH Enzyme Assay

Expression and purification of the enzyme was carried out following the protocol of DAS et al. (SAR-Based Optimization of a 4-Quinoline Carboxylic Acid Analogue with Potent Antiviral Activity. ACS Med. Chem. Lett. 2013, 4, 517-521). The prepared enzyme stock solution (325 μM) was thawed to 0 °C and carefully diluted to 87.5 nM with ice-cooled Tris-HCl buffer (50 mM Tris-HCl, 0.1 % Triton X-100, pH 8.0)). Stock solutions of all test compounds (100 mM), L-DHO (250 mM) and CoQ1 (250 mM) were prepared in DMSO, stored at -20 °C and thawed to room temperature before use. The reaction was performed in triplicates in a 96 well plate with total volumes of 200 μL containing L-dihydroorotate (L-DHO; 0.10 mM), coenzyme Q1 (CoQ1; 25 μM), 2,6-dichlorophenolindophenol (DCPIP; 60 μM), HsDHODH29-396 (35 nM) and the test compound (varying concentrations) in Tris-HCl buffer. Brequinar was assessed as reference inhibitor and a control without inhibitor but the equivalent amount of buffer instead was run for each test compound.

The compound stock solutions were diluted in Tris-HCl buffer to give nine different concentrations and each sample was treated with a freshly prepared substrate mixture containing L-DHO, CoQ1 and DCPIP. By simultaneous addition of 80 μL ice-cooled enzyme solution (87.5 μM) per well and immediate mixing by thorough pipetting, the reaction was started. The absorption of the mixture was monitored using a spectrophotometer (Berthold Technologies $TriStar_2S$ LB942 Multimode Reader, ICE software) with one data point per 10 seconds at an excitation wavelength of 600 nm and a temperature of 25 °C. During the first 200 s from enzyme addition, linear initial enzyme velocites were recorded, from which the IC50 values were calculated by non-linear fitting (Origin software) to the following equation:

$$v = \frac{V_0}{1 + \frac{c}{IC50}}$$

wherein

v is the determined initial, linear enzyme velocity,
$V_0$ is the calculated enzyme velocity without test compound and
c is the concentration of added test compound.

The obtained hDHODH inhibition data are shown in the table below:

| Parameter [nM] | Example | | | |
|---|---|---|---|---|
| | 1 | 2 | 3 | 4 |
| $IC_{50}$ | 9.2 | 90.7 | ≈230 | 50 |

The compound of Example 18 showed a percentual inhibition of hDHODH at a 1 μM concentration of 91% and

an $IC_{50}$ of 81 nM.

Example 21: lymphocyte inhibition

Lymphocyte inhibition was determined by using human PBMCs (peripheral blood mononuclear cells) as cell culture. The inhibitor compound was added at 8 different concentrations with each determination in triplicate and one control without inhibitor. T-cell stimulation was done by Cytostim, a reagent that binds T-cell receptors to MHC molecules of the antigen-presenting cells in an antibody-based manner and enables T-cell stimulation. Quantification was achieved by a flow cytometry-based method (Facs analysis) with staining of PBMCs with eFluor670 (eBioscience(TM)). The PBMCs are assigned to their populations (CD4/CD8 T cells) by use of other markers.

The compound of Example 1 showed an $IC_{50}$ of about 150 nM for CD4 and CD8 replication.

Example 22: ADME properties

Absorption, distribution, metabolism and excretion properties of selected inhibitors were determined.

**S9 Metabolism assay**

Stock solutions of all test compounds (100 mM) were prepared in DMSO, stored at-20 °C and diluted in PB to 600 μM prior to the assay. Rat liver S9 fractions (SigmaAldrich, Lot # SLCC9026 and # SLCB2232) were combined, aliquoted (200 μL), stored at -80 °C and thawed to 0 °C before use. The reaction was performed in triplicates in eppendorf tubes with total volumes of 300 μL containing $MgCl_2$ (5 mM), NADPH (5 mM), rat S9 fraction (1 mg) and the test compound (100 μM) in phosphate buffer (38.5 mM $Na_2HPO_4$, 11.5 mM $KH_2PO_4$, pH 7.3). 7-Ethoxycoumarin was assessed as reference inhibitor and a control without inhibitor but an equivalent amount of buffer instead was run. The ice cooled S9 fraction was added to a solution of $MgCl_2$ and NADPH in PB and the resulting mixture was preincubated for 10 minutes at 37 °C in a shaker. Subsequently the reaction was started by addition of the test compound (50 μL), followed by threefold pipetting. After 0 h, 1 h and 3 h, samples of 50 μL were taken from the reaction, mixed with 150 μL ice-cooled methanol and centrifuged at 14000 rpm and 4 °C for 15 minutes. The supernatant was filtered with a syringe fiter, immediately quantified on HPLC and stored at -20 °C until LC/MS analysis.

**Octanol-Water Partition Coefficient (logD)**

Octanol and phosphate buffered saline (PBS, 137 mM NaCl, 2.7 mM KCl, 10 mM $Na_2HPO_4$, 2.0 mM $KH_2PO_4$, pH 7.4) were saturated with each other prior to the experiment. Stock solutions of all test compounds (100 mM) were prepared in DMSO, stored at - 20 °C and diluted with the saturated PBS to 100 μM prior to the experiment. The experiment was performed in duplicates in 2 mL HPLC-vials. 300 μL of the test compound solution (100 μM) was overlayered with 300 μL saturated octanol. The emulsions were vortexed for 1 min and shaken for 1 h at 180 rpm and 25 °C. After phase separation, the aqueous phases and the initial 100 μM compound solution were quantified on HPLC. Partition coefficients were calculated using the following equation:

$$\log D = \log\left(\frac{\text{area}_{\text{in}} - \text{area}_{\text{aq}}}{\text{area}_{\text{aq}}}\right)$$

wherein

$area_{in}$ is the area under the curve in the chromatogram obtained for the initial, 100 μM compound solution and $area_{aq}$ is the area under the curve in the chromatogram obtained for one separated aqueous phase.

**Determination of Membrane Permeability (PAMPA Assay)**

Stock solutions of all test compounds (100 mM) were prepared in DMSO, stored at -20 °C and diluted with phosphate buffered saline (PBS, 137 mM NaCl, 2.7 mM KCl, 10 mM $Na_2HPO_4$, 2.0 mM $KH_2PO_4$, pH 7.4) to 200 μM prior to the experiment. The assay was performed in triplicates in a 96 well Corning Gentest Precoated PAMPA Plate System, which was stored at -20 °C and thawed to room temperature for 1 h prior to the assay. Wells of the donor plate were filled with 300 μL of the compound solutions (200 μM) and 200 μL PBS were added to the wells of the receiver plate. Two controls without inhibitor but an equivalent amount of buffer instead was run. Bubble-free assembling of the two plates was followed by 5 h incubation at 25 °C. Finally, the plates were carefully separated from each other. 150 μL

of each well of both plates were transferred to black 96-well FluoroNunc™ plates and quantified on fluorescence spectrophotometer (Berthold Technologies TriStar$^2$S LB942 Multimode Reader, ICE software). Membrane Permeabilities were calculated using the following equation:

$$P_{pampa} = \frac{-\ln(1 - \frac{c_{acc}(t)}{c_{eq}})}{A_{perm} \cdot (\frac{1}{V_{don}} + \frac{1}{V_{acc}}) \cdot t}$$

wherein

$C_{acc}(t)$ is the compound concentration in acceptor well at time t,
$A_{perm}$ is the area of the membrane filter (0.3 $cm^2$),
$V_{don}$ is the donor well volume (0.3 mL),
$V_{acc}$ is the acceptor well volume (0.2 mL) and
$C_{eq}$ is the equilibrium concentration with $c_{eq} = \frac{c_{don}(t) \cdot V_{don} + c_{acc}(t) \cdot V_{acc}}{V_{don} + V_{acc}}$ where $c_{don}(t)$ is the compound concentration in donor well at time t.

The obtained results are shown in the following table.

| | Ex. 1 | Ex. 2 | Ex. 18 |
|---|---|---|---|
| **Metabolic stability [% remaining at 1h in rat S9 fraction]** | 68.2 | 92.0 | 89.6 |
| **Log D (PBS pH 7.4/ octanol)** | 2.98 | 1.96 | 0.32 |
| **Membrane permeability [cm/s]** | $5{,}74 \cdot 10^{-6}$ | $3{,}23 \cdot 10^{-6}$ | $6{,}84 \cdot 10^{-6}$ |

The above data show that the tested compounds have acceptable ADME properties that encourage further *in vivo* pharmacokinetic studies for the development of treatments of RNA virus diseases. In particular, the compounds showed a high metabolic stability.

While compounds having an amide bond, such as

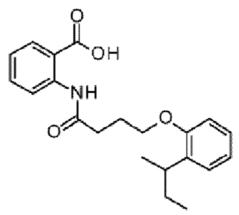

,

showed a cleavage of the amide bond (see hydrolysis study in S9 extract of rat liver shown in Figure 1; identified via LC-MS), the compounds of Examples 1 and 2 showed no amide cleavage (see Figure 2 (Example 1) and Figure 3 (Example 2)). Also the compound of Example 18 did not show amide cleavage.

## Claims

1. Compound, or a dimer or a pharmaceutically acceptable salt or solvate of said compound or dimer, for use in a method for the treatment of a disease, disorder or condition caused by an RNA virus, said compound having the general structure shown in Formula I:

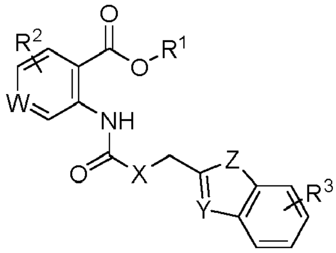

(I)

wherein:

W is C or N, and preferably is C;
$R^1$ is H, alkyl, cycloalkyl, heterocyclyl, -C(O)-alkyl or a pharmaceutically acceptable cation, wherein the alkyl or -C(O)-alkyl can be unsubstituted or optionally substituted with one or more moieties which can be the same or different, each moiety being independently selected from the group consisting of -OC(O)-alkyl, -OC(O)O-alkyl, heterocyclyl, aryl and heteroaryl,
or optionally the -C(O)-O-$R^1$-group is joined to the -NH-group or -X-group shown in Formula (I) to form together with the W-containing aromatic ring shown in Formula (I) a hetero ring system;
$R^2$ is one or more substituents independently selected from the group consisting of H, alkyl, alkenyl, alkynyl, aryl, alkoxy, aryloxy, halogen, haloalkyl, cycloalkyl, halocycloalkyl, heterocyclyl, hydroxyalkyl and -$NO_2$, wherein each of said alkyl, alkenyl, alkynyl, aryl, alkoxy, cycloalkyl, halocycloalkyl, heterocyclyl and aryloxy can be unsubstituted or optionally substituted with one or more moieties which can be the same or different, wherein each moiety is preferably independently selected from the group consisting of hydroxyl, halogen, alkyl, haloalkyl, aryl, haloaryl, alkylaryl, arylalkyl, cycloalkyl, aryloxy, alkoxy substituted with aryl, alkyl substituted with heterocyclyl, aryl substituted with haloaklkyl, aryl substituted with cycloalkyl, aryl substituted with arylalkyl, aryl substituted with alkoxy, aryl substituted with aryloxy, aryl substituted with -O-arylalkyl, aryl substituted with aryl, aryloxy, aryloxy substituted with alkyl, aryloxy substituted with cycloalkyl and aryloxy substituted with arylalkyl,
or optionally $R^2$ represents two substituents which are joined to form together with the aromatic ring shown in Formula I a substituted or unsubstituted ring or hetero ring system,
or optionally at least one of $R^2$ is joined to the -NH-group or -X-group shown in Formula (I) to form together with the W-containing aromatic ring shown in Formula (I) a hetero ring system;
$R^3$ is one or more substituents independently selected from the group consisting of H, alkyl, halogen, haloalkyl, cycloalkyl, halocycloalkyl, heterocyclyl, alkoxy, aryl and arylalkyl, wherein each of said alkyl, haloalkyl, cycloalkyl, halocycloalkyl, heterocyclyl, alkoxy, aryl and arylalkyl can be unsubstituted or optionally substituted with one or more moieties which can be the same or different, wherein each moiety is preferably independently selected from the group consisting of hydroxyl, halogen, alkyl, haloalkyl, aryl, haloaryl, alkylaryl, arylalkyl, cycloalkyl, aryloxy, alkoxy substituted with aryl, alkyl substituted with heterocyclyl, aryl substituted with haloaklkyl, aryl substituted with cycloalkyl, aryl substituted with arylalkyl, aryl substituted with alkoxy, aryl substituted with aryloxy, aryl substituted with -O-arylalkyl, aryl substituted with aryl, aryloxy, aryloxy substituted with alkyl, aryloxy substituted with cycloalkyl and aryloxy substituted with arylalkyl;
X is O or NH, and preferably is NH;
Y is $CR^4$ or N, and preferably is $CR^4$;
Z is O, S, NH or $CR^5_2$, and preferably is O;
$R^4$ is H, halogen, alkyl, haloalkyl, cycloalkyl, halocycloalkyl, heterocyclyl, alkoxy or acetyl, and preferably is H; and
$R^5$ is independently selected from the group consisting of H, halogen, alkyl, haloalkyl, cycloalkyl, halocycloalkyl, heterocyclyl, alkoxy and acetyl, and preferably is H.

**2.** Compound, or a dimer or a pharmaceutically acceptable salt or solvate of said compound or dimer, for use according to claim 1, wherein $R^2$ is H.

**3.** Compound, or a dimer or a pharmaceutically acceptable salt or solvate of said compound or dimer, for use according to claim 1 or 2, wherein Y is $CR^4$ and $R^4$ is H.

**4.** Compound, or a dimer or a pharmaceutically acceptable salt or solvate of said compound or dimer, for use according to anyone of claims 1 to 3, wherein
$R^3$ is one or more substituents independently selected from the group consisting of H, alkyl, halogen and haloalkyl.

**5.** Compound, or a dimer or a pharmaceutically acceptable salt or solvate of said compound or dimer, for use according to claim 4, wherein the alkyl is 2-methylbutan-2-yl, the halogen is F, and/or the haloalkyl is trifluoromethyl.

**6.** Compound, or a dimer or a pharmaceutically acceptable salt or solvate of said compound or dimer, for use according to anyone of claims 1 to 4, said compound having the general structure shown in Formula Ia or Ib:

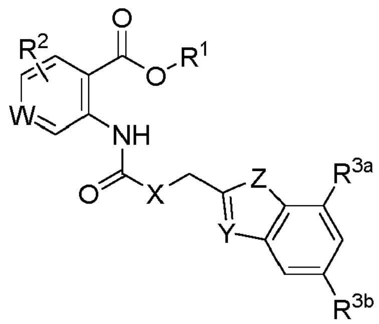
(Ia)

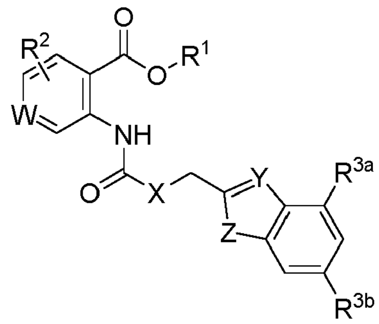
(Ib)

wherein:

$R^{3a}$ is alkyl or haloalkyl; and
$R^{3b}$ is H or halogen, wherein the halogen is preferably F.

**7.** Compound, or a dimer or a pharmaceutically acceptable salt or solvate of said compound or dimer, for use according to claim 6, wherein the alkyl is 2-methylbutan-2-yl and the haloalkyl is trifluoromethyl.

**8.** Compound, or a dimer or a pharmaceutically acceptable salt or solvate of said compound or dimer, for use according to anyone of claims 1 to 7, having the following formula:

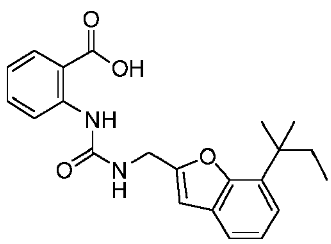
,
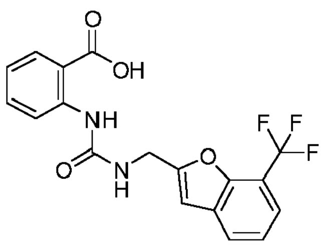
or
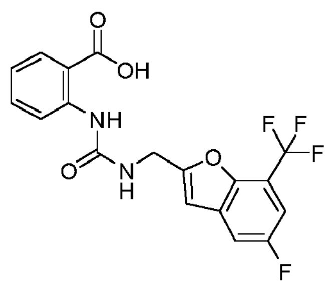
.

**9.** Compound, or a dimer or a pharmaceutically acceptable salt or solvate of said compound or dimer, said compound having the general structure shown in Formula I:

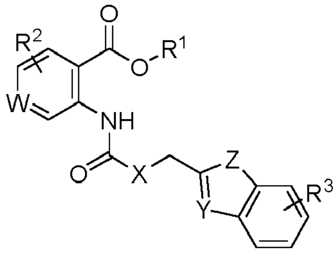

(I)

wherein:

W is C or N, and preferably is C;
$R^1$ is H, alkyl, cycloalkyl, heterocyclyl, -C(O)-alkyl or a pharmaceutically acceptable cation, wherein the alkyl or -C(0)-alkyl can be unsubstituted or optionally substituted with one or more moieties which can be the same or different, each moiety being independently selected from the group consisting of -OC(O)-alkyl, -OC(O)O-alkyl, heterocyclyl, aryl and heteroaryl,
or optionally the -C(O)-O-$R^1$-group is joined to the -NH-group or -X-group shown in Formula (I) to form together with the W-containing aromatic ring shown in Formula (I) a hetero ring system;
$R^2$ is one or more substituents independently selected from the group consisting of H, alkyl, alkenyl, alkynyl, aryl, alkoxy, aryloxy, halogen, haloalkyl, cycloalkyl, halocycloalkyl, heterocyclyl, hydroxyalkyl and -$NO_2$, wherein each of said alkyl, alkenyl, alkynyl, aryl, alkoxy, cycloalkyl, halocycloalkyl, heterocyclyl and aryloxy can be unsubstituted or optionally substituted with one or more moieties which can be the same or different, wherein each moiety is preferably independently selected from the group consisting of hydroxyl, halogen, alkyl, haloalkyl, aryl, haloaryl, alkylaryl, arylalkyl, cycloalkyl, aryloxy, alkoxy substituted with aryl, alkyl substituted with heterocyclyl, aryl substituted with haloaklkyl, aryl substituted with cycloalkyl, aryl substituted with arylalkyl, aryl substituted with alkoxy, aryl substituted with aryloxy, aryl substituted with -O-arylalkyl, aryl substituted with aryl, aryloxy, aryloxy substituted with alkyl, aryloxy substituted with cycloalkyl and aryloxy substituted with arylalkyl,
or optionally $R^2$ represents two substituents which are joined to form together with the aromatic ring shown in Formula I a substituted or unsubstituted ring or hetero ring system,
or optionally at least one of $R^2$ is joined to the -NH-group or -X-group shown in Formula (I) to form together with the W-containing aromatic ring shown in Formula (I) a hetero ring system;
$R^3$ is one or more substituents independently selected from the group consisting of H, alkyl, halogen, haloalkyl, cycloalkyl, halocycloalkyl, heterocyclyl, alkoxy, aryl and arylalkyl, wherein each of said alkyl, haloalkyl, cycloalkyl, halocycloalkyl, heterocyclyl, alkoxy, aryl and arylalkyl can be unsubstituted or optionally substituted with one or more moieties which can be the same or different, wherein each moiety is preferably independently selected from the group consisting of hydroxyl, halogen, alkyl, haloalkyl, aryl, haloaryl, alkylaryl, arylalkyl, cycloalkyl, aryloxy, alkoxy substituted with aryl, alkyl substituted with heterocyclyl, aryl substituted with haloaklkyl, aryl substituted with cycloalkyl, aryl substituted with arylalkyl, aryl substituted with alkoxy, aryl substituted with aryloxy, aryl substituted with -O-arylalkyl, aryl substituted with aryl, aryloxy, aryloxy substituted with alkyl, aryloxy substituted with cycloalkyl and aryloxy substituted with arylalkyl;
X is O or NH, and preferably is NH;
Y is $CR^4$ or N, and preferably is $CR^4$;
Z is O, S, NH or $CR^5_2$, and preferably is O;
$R^4$ is H, halogen, alkyl, haloalkyl, cycloalkyl, halocycloalkyl, heterocyclyl, alkoxy or acetyl, and preferably is H; and
$R^5$ is independently selected from the group consisting of H, halogen, alkyl, haloalkyl, cycloalkyl, halocycloalkyl, heterocyclyl, alkoxy and acetyl, and preferably is H.

**10.** Compound, or a dimer or a pharmaceutically acceptable salt or solvate of said compound or dimer, for use in a method for the treatment of a disease, disorder or condition caused by an RNA virus, said compound having the general structure shown in Formula II:

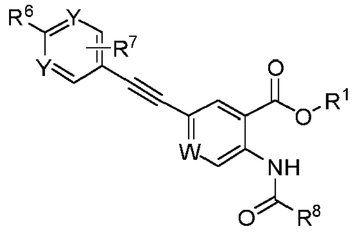

(II)

wherein:

W is C or N, and preferably is C;
$R^1$ is H, alkyl, cycloalkyl, heterocyclyl, -C(O)-alkyl or a pharmaceutically acceptable cation, wherein the alkyl or -C(0)-alkyl can be unsubstituted or optionally substituted with one or more moieties which can be the same or different, each moiety being independently selected from the group consisting of -OC(O)-alkyl, -OC(O)O-alkyl, heterocyclyl, aryl and heteroaryl;
Y is independently selected from $CR^4$ and N, and preferably is $CR^4$;
$R^4$ is H, halogen, alkyl, haloalkyl, cycloalkyl, halocycloalkyl, heterocyclyl, alkoxy or acetyl, and preferably is H;
$R^6$ is heterocyclyl or heterocyclylalkyl, wherein the heterocyclyl and heterocyclylalkyl can be unsubstituted or substituted with one or more ring system substituents which can be the same or different ;
$R^7$ is one or more substituents independently selected from the group consisting of H, halogen, alkyl, haloalkyl, alkoxy, -C(O)-alkyl, -C(O)-haloalkyl, $-NH_2$, -NH-alkyl and $-N(alkyl)_2$; and
$R^8$ is -C(O)-alkyl, -C(O)O-alkyl, -C(O)NH-alkyl, -C(O)-cycloalkyl, -C(O)O-cycloalkyl, -C(O)NH-cycloalkyl, -C(O)-aryl, -C(O)O-aryl, -C(O)NH-aryl, -C(O)-heteroaryl, -C(O)O-heteroaryl, -C(O)NH-heteroaryl, aryl substituted with $R^{10}$, heteroaryl substituted with $R^{10}$, $-S(O_2)-R^{11}$ or

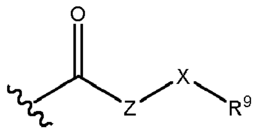

;

wherein
Z is $-(CH_2)_n-$, $-O-(CH_2)_n-$, $-NH-(CH_2)_n-$, $-(CH_2)_p-L-(CH_2)_q-$, $-O-(CH_2)_p-L-(CH_2)_q-$ or $-NH-(CH_2)_p-L-(CH_2)_q-$,
n is an integer from 1 to 6;
p and q are integers independently selected from 0 to 6;
L is a linking group selected from the group consisting of heteroaryl, aryl, heterocyclyl and cycloalkyl;
X is O, S, NH, $CH_2$, S(O) or C(O), and preferably is O, S or NH;
$R^9$ is aryl or heteroaryl, wherein said aryl or heteroaryl can be unsubstituted or optionally substituted with one or more moieties which can be the same or different, each moiety being independently selected from the group consisting of H, halogen, alkyl, cycloalkyl, heterocyclyl, aryl, heteroaryl, alkoxy, aryloxy, arylalkyl, alkylaryl, haloaryl, haloalkylaryl, haloalkyl and trialkylsilyl;
$R^{10}$ is aryloxy or arylalkyl or optionally $R^{10}$ represents two substituents linked to each other to form together with the aryl or heteroaryl a polycyclic ring system, wherein preferably the polycyclic ring system is a naphthalene or fluorene; and
$R^{11}$ is alkyl, cycloalkyl or $-Z-X-R^9$.

**11.** Compound, or a dimer or a pharmaceutically acceptable salt or solvate of said compound or dimer, for use according to claim 10, wherein $R^6$ is selected from the group consisting of

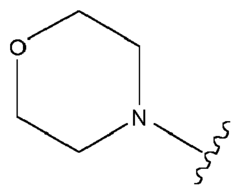

,

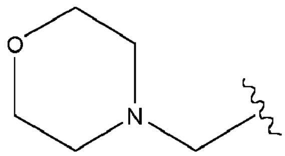
,

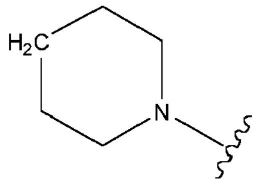
,

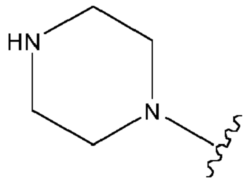
,

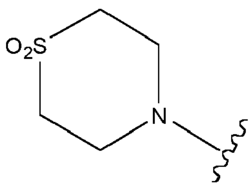
,

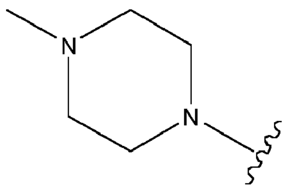

and

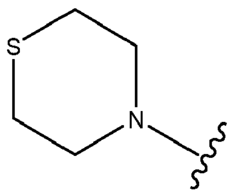
,

and preferably is

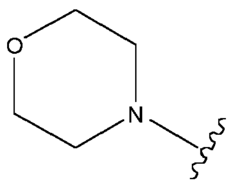
.

**12.** Compound, or a dimer or a pharmaceutically acceptable salt or solvate of said compound or dimer, for use according

to anyone of claims 10 to 11, having the following formula:

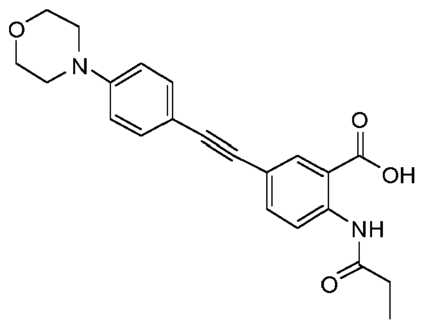 ,

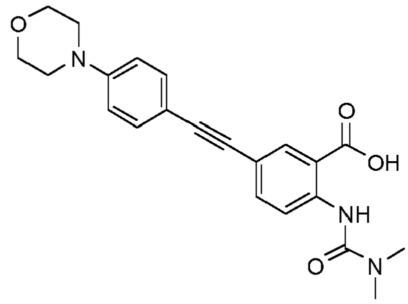 ,

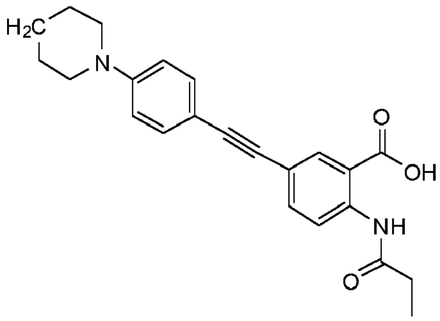 ,

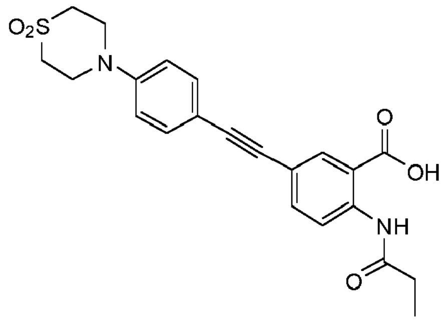 ,

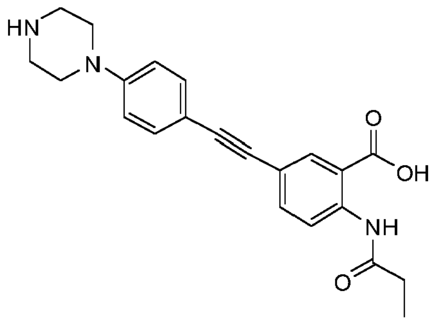 ,

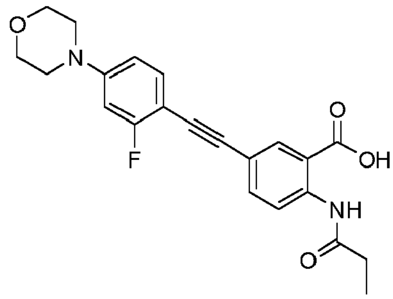 ,

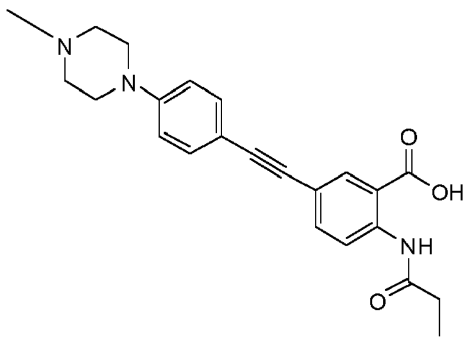 ,

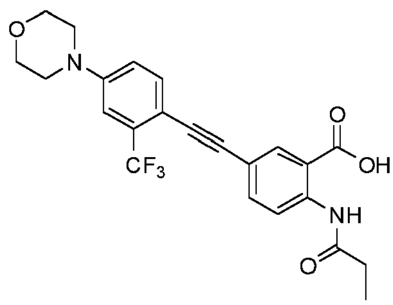 ,

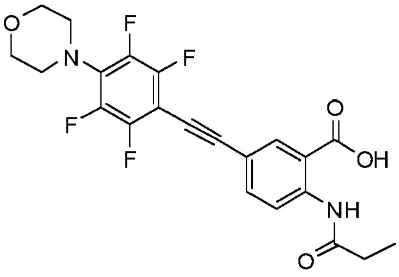,

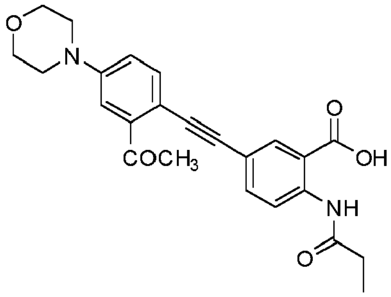,

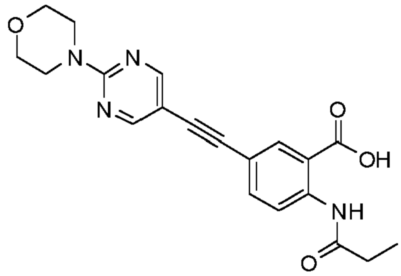,

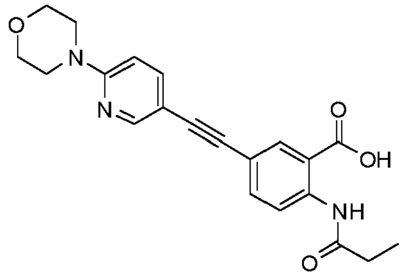

or

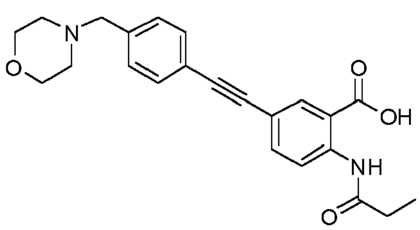.

**13.** Compound, or a dimer or a pharmaceutically acceptable salt or solvate of said compound or dimer, said compound having the general structure shown in Formula II:

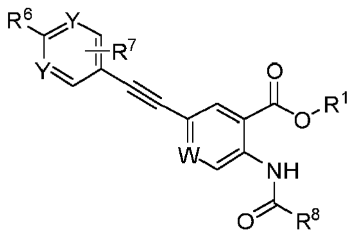

(II)

wherein:

W is C or N, and preferably is C;

$R^1$ is H, alkyl, cycloalkyl, heterocyclyl, -C(O)-alkyl or a pharmaceutically acceptable cation, wherein the alkyl or -C(O)-alkyl can be unsubstituted or optionally substituted with one or more moieties which can be the same or different, each moiety being independently selected from the group consisting of -OC(O)-alkyl, -OC(O)O-alkyl, heterocyclyl, aryl and heteroaryl;

Y is independently selected from $CR^4$ and N, and preferably is $CR^4$;

$R^4$ is H, halogen, alkyl, haloalkyl, cycloalkyl, halocycloalkyl, heterocyclyl, alkoxy or acetyl, and preferably is H;

$R^6$ is heterocyclyl or heterocyclylalkyl, wherein the heterocyclyl and heterocyclylalkyl can be unsubstituted or substituted with one or more ring system substituents which can be the same or different ;

$R^7$ is one or more substituents independently selected from the group consisting of H, halogen, alkyl, haloalkyl, alkoxy, -C(O)-alkyl, -C(O)-haloalkyl, $-NH_2$, -NH-alkyl and $-N(alkyl)_2$; and

$R^8$ is-C(O)-alkyl, -C(O)O-alkyl, -C(O)NH-alkyl, -C(O)-cycloalkyl, -C(O)O-cycloalkyl, -C(O)NH-cycloalkyl, -C(O)-aryl, -C(O)O-aryl, -C(O)NH-aryl, -C(O)-heteroaryl, -C(O)O-heteroaryl, -C(O)NH-heteroaryl, aryl substituted with $R^{10}$, heteroaryl substituted with $R^{10}$, -$S(O_2)$-$R^{11}$ or

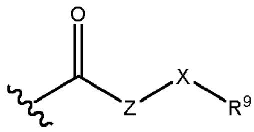;

wherein
Z is $-(CH_2)_n-$, $-O-(CH_2)_n-$, $-NH-(CH_2)_n-$, $-(CH_2)_p-L-(CH_2)_q-$, $-O-(CH_2)_p-L-(CH_2)_q-$ or $-NH-(CH_2)_p-L-(CH_2)_q-$,
n is an integer from 1 to 6;
p and q are integers independently selected from 0 to 6;
L is a linking group selected from the group consisting of heteroaryl, aryl, heterocyclyl and cycloalkyl;
X is O, S, NH, $CH_2$, S(O) or C(O), and preferably is O, S or NH;
$R^9$ is aryl or heteroaryl, wherein said aryl or heteroaryl can be unsubstituted or optionally substituted with one or more moieties which can be the same or different, each moiety being independently selected from the group consisting of H, halogen, alkyl, cycloalkyl, heterocyclyl, aryl, heteroaryl, alkoxy, aryloxy, arylalkyl, alkylaryl, haloaryl, haloalkylaryl, haloalkyl and trialkylsilyl;
$R^{10}$ is aryloxy or arylalkyl or optionally $R^{10}$ represents two substituents linked to each other to form together with the aryl or heteroaryl a polycyclic ring system, wherein preferably the polycyclic ring system is a naphthalene or fluorene; and
$R^{11}$ is alkyl, cycloalkyl or -Z-X-$R^9$.

**14.** Compound, or a dimer or a pharmaceutically acceptable salt or solvate of said compound or dimer, for use in a method for the treatment of a disease, disorder or condition caused by an RNA virus, said compound having the general structure shown in Formula III:

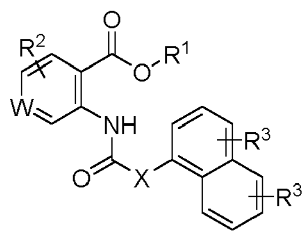

(III)

wherein:

W is N;
$R^1$ is H, alkyl, cycloalkyl, heterocyclyl, -C(O)-alkyl or a pharmaceutically acceptable cation, wherein the alkyl or -C(O)-alkyl can be unsubstituted or optionally substituted with one or more moieties which can be the same or different, each moiety being independently selected from the group consisting of -OC(O)-alkyl, -OC(O)O-alkyl, heterocyclyl, aryl and heteroaryl,
or optionally the -C(O)-O-$R^1$-group is joined to the -NH-group or -X-group shown in Formula (III) to form together with the W-containing aromatic ring shown in Formula (III) a hetero ring system;
$R^2$ is one or more substituents independently selected from the group consisting of H, alkyl, alkenyl, alkynyl, aryl, alkoxy, aryloxy, halogen, haloalkyl, cycloalkyl, halocycloalkyl, heterocyclyl, hydroxyalkyl and $-NO_2$, wherein each of said alkyl, alkenyl, alkynyl, aryl, alkoxy, cycloalkyl, halocycloalkyl, heterocyclyl and aryloxy can be unsubstituted or optionally substituted with one or more moieties which can be the same or different, wherein each moiety is preferably independently selected from the group consisting of hydroxyl, halogen, alkyl, haloalkyl, aryl, haloaryl, alkylaryl, arylalkyl, cycloalkyl, aryloxy, alkoxy substituted with aryl, alkyl substituted with heterocyclyl, aryl substituted with haloaklkyl, aryl substituted with cycloalkyl, aryl substituted with arylalkyl, aryl substituted with alkoxy, aryl substituted with aryloxy, aryl substituted with -O-arylalkyl, aryl substituted with aryl, aryloxy, aryloxy substituted with alkyl, aryloxy substituted with cycloalkyl and aryloxy substituted with arylalkyl, or optionally $R^2$ represents two substituents which are joined to form together with the aromatic ring shown in Formula III a substituted or unsubstituted ring or hetero ring system,

or optionally at least one of $R^2$ is joined to the -NH-group or -X-group shown in Formula (III) to form together with the W-containing aromatic ring shown in Formula (III) a hetero ring system;

X is $CH_2$, O or NH, and preferably is NH; and

$R^3$ is one or more substituents independently selected from the group consisting of H, alkyl, halogen, haloalkyl, cycloalkyl, halocycloalkyl, heterocyclyl, alkoxy, aryl, arylalkyl, heteroalkyl, heteroarylalkyl, amido, carbamoyl, wherein each of said alkyl, haloalkyl, cycloalkyl, halocycloalkyl, heterocyclyl, alkoxy, aryl, arylalkyl, heteroalkyl, heteroarylalkyl, amido, and carbamoyl can be unsubstituted or optionally substituted with one or more moieties which can be the same or different, wherein each moiety is preferably independently selected from the group consisting of hydroxyl, halogen, alkyl, haloalkyl, aryl, haloaryl, alkylaryl, arylalkyl, cycloalkyl, aryloxy, alkoxy substituted with aryl, alkyl substituted with heterocyclyl, aryl substituted with haloaklkyl, aryl substituted with cycloalkyl, aryl substituted with arylalkyl, aryl substituted with alkoxy, aryl substituted with aryloxy, aryl substituted with -O-arylalkyl, aryl substituted with aryl, aryloxy, aryloxy substituted with alkyl, aryloxy substituted with cycloalkyl and aryloxy substituted with arylalkyl;

or optionally $R^3$ represents two substituents which are joined to form together with the naphthyl ring system shown in Formula III a substituted or unsubstituted ring or hetero ring system.

**15.** Compound, or a dimer or a pharmaceutically acceptable salt or solvate of said compound or dimer, for use according to anyone of claims 1 to 8, 10 to 12 and 14, wherein the RNA virus is selected form the group consisting of bunya viruses including Toscana virus (TOSV), hazara virus (HAZV), tahyna virus (TAHV), rift valley fever virus (RVFV), Lassa virus (LSAV), Punta Toro phlebovirus (PTV) and Crimean-Congo hemorrhagic fever orthonairovirus (CCHFV); flavi viruses including yellow fever virus (YFV), dengue virus (DENV), tick-borne encephalitis virus (TBEV), zika virus (ZIKV) and Hepatitis C virus (HCV); toga viruses including venezuelan equine encephalitis virus (VEEV), Sindbis virus (SINV) and Chikungunya virus (CHIKV); mononegaviruses including Ebola virus (EBOV), Marburg virus (MARV), Human parainfluenza virus 3 (HPIV-3), Nipah virus (NiV) and Vesicular stomatitis virus (VSV); picorna viruses including coxsackievirus (CV); nidoviruses including corona viruses such as Severe acute respiratory syndrome-related coronavirus (SARS-CoV), Severe acute respiratory syndrome-related coronavirus 2 (SARS-CoV-2) and Middle-East respiratory syndrome-related coronavirus (MERS-CoV); influenza viruses and reoviruses including reovirus type 1 (Reo-1).

Figure 1:

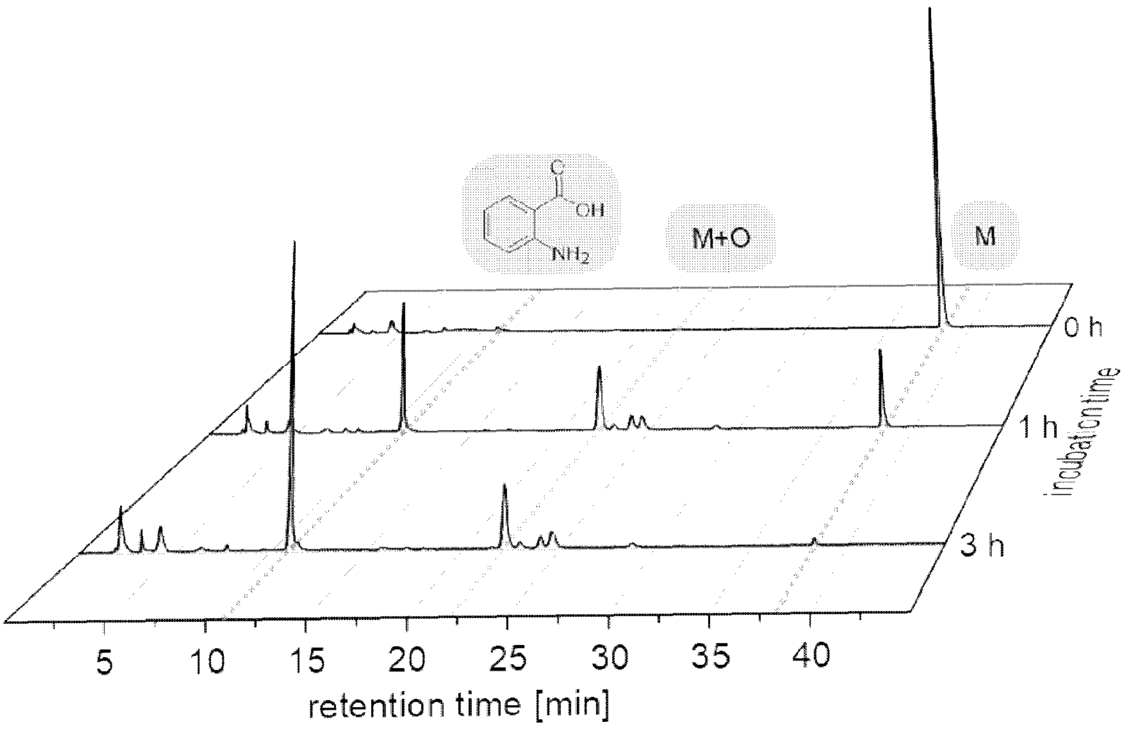
M+O
M
0 h
1 h
3 h
incubation time
5
10
15
20
25
30
35
40
retention time [min]

Figure 2:

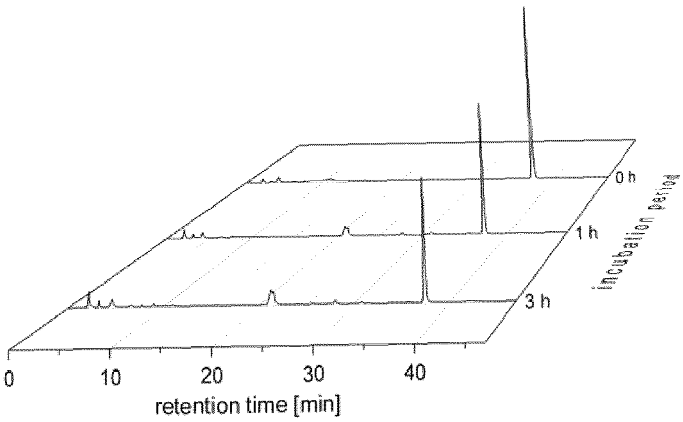

Figure 3:

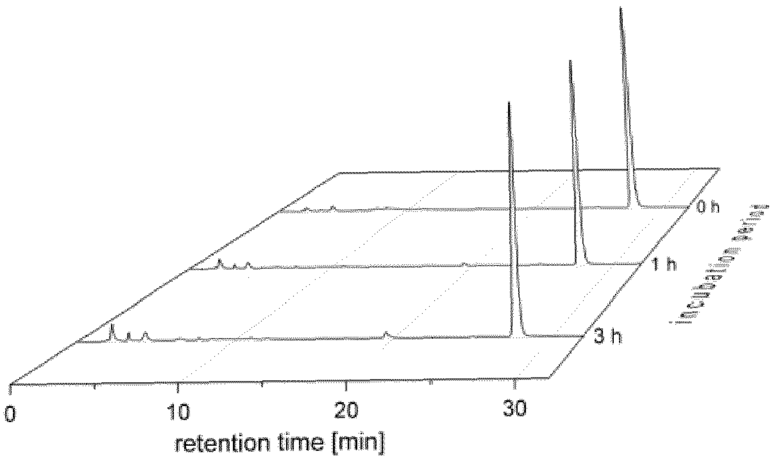

Europäisches Patentamt
European Patent Office
Office européen des brevets

# EUROPEAN SEARCH REPORT

Application Number
EP 21 18 5175

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | WO 2020/225330 A1 (UNIV HAMBURG [DE]; AIX MARSEILLE UNIV [FR]) 12 November 2020 (2020-11-12) * the whole document * | 14,15 | INV. A61K31/343 A61K31/4418 A61K31/451 A61K31/495 A61K31/5375 A61K31/54 A61P31/14 |
| X | WO 02/070500 A1 (HOFFMANN LA ROCHE [CH]) 12 September 2002 (2002-09-12) * the whole document * * examples 2-4 * | 9 | |
| X | WO 02/070514 A1 (HOFFMANN LA ROCHE [CH]) 12 September 2002 (2002-09-12) * the whole document * * example 3 * | 9 | |
| A | WO 2013/143597 A1 (GLAXO GROUP LTD [GB]; BARKER MICHAEL DAVID [GB] ET AL.) 3 October 2013 (2013-10-03) * the whole document * * page 50 * * page 68; compound 8 * | 14 | TECHNICAL FIELDS SEARCHED (IPC) |
| Y,D | US 2014/080768 A1 (PALMER ALAN [GB] ET AL) 20 March 2014 (2014-03-20) * the whole document * | 1-15 | A61K A61P |
| Y | US 2010/280081 A1 (VITT DANIEL [DE] ET AL) 4 November 2010 (2010-11-04) * the whole document * * compounds 7, 18-20 * | 1-15 | |

-/--

1 The present search report has been drawn up for all claims

| Place of search | Date of completion of the search | Examiner |
|---|---|---|
| Munich | 20 December 2021 | Houyvet-Landriscina |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 21 18 5175

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | MUNIER-LEHMANN HÉLÈNE ET AL: "On dihydroorotate dehydrogenases and their inhibitors and uses.", JOURNAL OF MEDICINAL CHEMISTRY 25 APR 2013, vol. 56, no. 8, 25 April 2013 (2013-04-25), pages 3148-3167, XP055148746, ISSN: 1520-4804 * the whole document * * figures 7-9, 11 * ----- | 1-15 | |
| Y | HAHN FRIEDRICH ET AL: "IMU-838, a Developmental DHODH Inhibitor in Phase II for Autoimmune Disease, Shows Anti-SARS-CoV-2 and Broad-Spectrum Antiviral Efficacy In Vitro.", VIRUSES 05 12 2020, vol. 12, no. 12, 5 December 2020 (2020-12-05), XP055813677, ISSN: 1999-4915 * the whole document * ----- | 1-15 | TECHNICAL FIELDS SEARCHED (IPC) |

1 The present search report has been drawn up for all claims

| Place of search | Date of completion of the search | Examiner |
|---|---|---|
| Munich | 20 December 2021 | Houyvet-Landriscina |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 2

Europäisches Patentamt
European Patent Office
Office européen des brevets

Application Number
EP 21 18 5175

## CLAIMS INCURRING FEES

The present European patent application comprised at the time of filing claims for which payment was due.

- [ ] Only part of the claims have been paid within the prescribed time limit. The present European search report has been drawn up for those claims for which no payment was due and for those claims for which claims fees have been paid, namely claim(s):

- [ ] No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

- [ ] All further search fees have been paid within the fixed time limit. The present European search report has been drawn up for all claims.

- [x] As all searchable claims could be searched without effort justifying an additional fee, the Search Division did not invite payment of any additional fee.

- [ ] Only part of the further search fees have been paid within the fixed time limit. The present European search report has been drawn up for those parts of the European patent application which relate to the inventions in respect of which search fees have been paid, namely claims:

- [ ] None of the further search fees have been paid within the fixed time limit. The present European search report has been drawn up for those parts of the European patent application which relate to the invention first mentioned in the claims, namely claims:

- [ ] The present supplementary European search report has been drawn up for those parts of the European patent application which relate to the invention first mentioned in the claims (Rule 164 (1) EPC).

Europäisches Patentamt
European Patent Office
Office européen des brevets

# LACK OF UNITY OF INVENTION
# SHEET B

Application Number
EP 21 18 5175

The Search Division considers that the present European patent application does not comply with the requirements of unity of invention and relates to several inventions or groups of inventions, namely:

1. claims: 1-15

Anthranilic acid derivatives for use in the treatment of RNA viruses

1.1. claims: 1-9(completely); 15(partially)

Compound of formula (I) and use thereof in the treatment of RNA viruses

1.2. claims: 10-13(completely); 15(partially)

Compound of formula (II) and use thereof in the treatment of RNA viruses.

1.3. claims: 14(completely); 15(partially)

Compound of formula (III) for use in the treatment of RNA viruses

---

Please note that all inventions mentioned under item 1, although not necessarily linked by a common inventive concept, could be searched without effort justifying an additional fee.

## ANNEX TO THE EUROPEAN SEARCH REPORT ON EUROPEAN PATENT APPLICATION NO. EP 21 18 5175

This annex lists the patent family members relating to the patent documents cited in the above-mentioned European search report. The members are as contained in the European Patent Office EDP file on
The European Patent Office is in no way liable for these particulars which are merely given for the purpose of information.

20-12-2021

| Patent document cited in search report | Publication date | Patent family member(s) | Publication date |
|---|---|---|---|
| WO 2020225330 A1 | 12-11-2020 | NONE | |
| WO 02070500 A1 | 12-09-2002 | AR 033430 A1 | 17-12-2003 |
| | | AT 309994 T | 15-12-2005 |
| | | AU 2002253061 B2 | 07-04-2005 |
| | | BR 0207795 A | 23-03-2004 |
| | | CA 2438737 A1 | 12-09-2002 |
| | | CN 1527824 A | 08-09-2004 |
| | | DE 60207399 T2 | 03-08-2006 |
| | | EP 1379516 A1 | 14-01-2004 |
| | | ES 2252443 T3 | 16-05-2006 |
| | | JP 4018985 B2 | 05-12-2007 |
| | | JP 2004529899 A | 30-09-2004 |
| | | KR 20040023591 A | 18-03-2004 |
| | | MX PA03007865 A | 04-12-2003 |
| | | PA 8540601 A1 | 30-09-2002 |
| | | PE 20020905 A1 | 19-10-2002 |
| | | US 2002165235 A1 | 07-11-2002 |
| | | WO 02070500 A1 | 12-09-2002 |
| WO 02070514 A1 | 12-09-2002 | AR 033429 A1 | 17-12-2003 |
| | | AT 321041 T | 15-04-2006 |
| | | BR 0207730 A | 23-03-2004 |
| | | CA 2438813 A1 | 12-09-2002 |
| | | CN 1494544 A | 05-05-2004 |
| | | DE 60210058 T2 | 09-11-2006 |
| | | EP 1368345 A1 | 10-12-2003 |
| | | ES 2260425 T3 | 01-11-2006 |
| | | JP 2004526721 A | 02-09-2004 |
| | | KR 20030078962 A | 08-10-2003 |
| | | MX PA03007864 A | 04-12-2003 |
| | | US 2002169171 A1 | 14-11-2002 |
| | | WO 02070514 A1 | 12-09-2002 |
| | | ZA 200306283 B | 15-11-2004 |
| WO 2013143597 A1 | 03-10-2013 | NONE | |
| US 2014080768 A1 | 20-03-2014 | NONE | |
| US 2010280081 A1 | 04-11-2010 | AR 076837 A1 | 13-07-2011 |
| | | AU 2010244462 A1 | 17-11-2011 |
| | | CA 2761130 A1 | 11-11-2010 |
| | | CN 102458389 A | 16-05-2012 |
| | | CO 6450622 A2 | 31-05-2012 |
| | | EP 2427186 A2 | 14-03-2012 |
| | | NZ 596014 A | 28-06-2013 |

EPO FORM P0459

For more details about this annex : see Official Journal of the European Patent Office, No. 12/82

# ANNEX TO THE EUROPEAN SEARCH REPORT ON EUROPEAN PATENT APPLICATION NO.

EP 21 18 5175

This annex lists the patent family members relating to the patent documents cited in the above-mentioned European search report. The members are as contained in the European Patent Office EDP file on
The European Patent Office is in no way liable for these particulars which are merely given for the purpose of information.

20-12-2021

| Patent document cited in search report | Publication date | Patent family member(s) | Publication date |
|---|---|---|---|
| | | PE 20120898 A1 | 24-08-2012 |
| | | SG 175854 A1 | 29-12-2011 |
| | | TW 201043228 A | 16-12-2010 |
| | | US 2010280081 A1 | 04-11-2010 |
| | | UY 32605 A | 31-12-2010 |
| | | WO 2010128050 A2 | 11-11-2010 |
| | | ZA 201108131 B | 25-07-2012 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2005075410 A
- US 20140080768 A1
- WO 2009153043 A1
- WO 2020225330 A


### Non-patent literature cited in the description


- **MUNIER-LEHMANN et al.** *J. Med. Chem.,* 2013, vol. 56, 3148-3167
- **CHEN et al.** *Transplant Immunology,* 2010, vol. 23, 180-184
- **MARSCHALL et al.** *Antiviral Res.,* 2013, vol. 100, 640-648
- **WANG et al.** *J. Virol.,* 2011, vol. 85, 6548-6556
- **ÖLSCHLÄGER et al.** Complete sequence and phylogenetic characterisation of Crimean-Congo hemorrhagic fever virus from Afghanistan. *J. Clin. Virol.,* 2011, vol. 50, 90-92
- **LECOMPTE et al.** J. Mastomys natalensis and Lassa fever, West Africa. *Emerg. Infect. Dis.,* 2006, vol. 12, 1971-1974
- **GÜNTHER.** Application of real-time PCR for testing antiviral compounds against Lassa virus, SARS coronavirus and Ebola virus in vitro. *Antiviral Res.,* 2004, vol. 63, 209-215
- **DAS et al.** SAR-Based Optimization of a 4-Quinoline Carboxylic Acid Analogue with Potent Antiviral Activity. *ACS Med. Chem. Lett.,* 2013, vol. 4, 517-521